# EUROPEAN PATENT APPLICATION

(11) **EP 3 797 781 A1**
(43) Date of publication of application: **31.03.2021**
(21) Application number: 19382832.4
(22) Date of filing: 26.09.2019
(51) Int. Cl.: A61K 31/704, A61K 31/519, A61K 31/7076, A61P 35/02

(54) **USE OF EC-7072 AND SYNERGIC COMPOSITIONS THEREOF IN TREATMENT OF CHRONIC LYMPHOCYTIC LEUKEMIA**

(71) Applicant: EntreChem, S.L., 33011 Oviedo (ES)
(72) Inventor: Lorenzo Herrero, Seila, 33006 Oviedo (ES); López Soto, Alejandro, 33006 Oviedo (ES); González Rodríguez, Segundo, 33006 Oviedo (ES); Sordo Bahamonde, Christian, 33006 Oviedo (ES); Morís Varas, Francisco, 33011 Oviedo (ES); Núñez González, Luz Elena, 33011 Oviedo (ES); Pérez Escuredo, Jhudit, 33011 Oviedo (ES)
(74) Representative: Manuel Illescas y Asociados, S.L.

(57) **Abstract**

The present invention is focused on a compound of Formula (I), 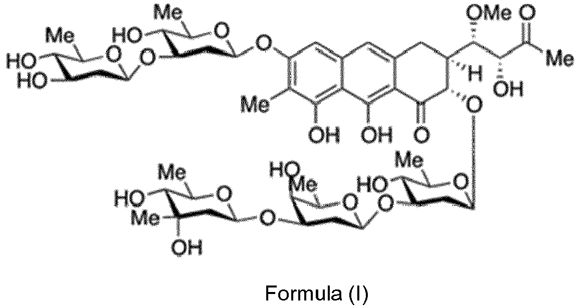 or a pharmaceutically acceptable salt, co-crystal or solvate thereof, for use in treatment of chronic lymphocytic leukemia, as well as a composition comprising:
(a) said compound of Formula (I), or a pharmaceutically acceptable salt, co-crystal or solvate thereof; and
(b) at least one chemotherapeutic agent selected from idelalisib or fludarabine, or a salt, co-crystal or solvate thereof.

SEQUENCE LISTING

<110> EntreChem S.L.

<120> Use of EC-7072 and synergic compositions thereof in treatment of
chronic lymphocytic leukemia

<130> EP-08213

<160> 24

<170> BiSSAP 1.3.6

<210> 1
<211> 21
<212> DNA
<213> Artificial Sequence

<220>
<223> CD79B forward primer

<400> 1
ccaggctggc gttgtctcct g                                                21

<210> 2
<211> 22
<212> DNA
<213> Artificial Sequence

<220>
<223> CD79B reverse primer

<400> 2
ggtaccggtc ctccgatctg gc                                               22

<210> 3
<211> 21
<212> DNA
<213> Artificial Sequence

<220>
<223> SYK forward primer

<400> 3
tgcactatcg catcgacaaa g                                                21

<210> 4
<211> 21
<212> DNA
<213> Artificial Sequence

<220>
<223> SYK reverse primer

<400> 4
catttccctg tgtgccgatt t                                                21

<210> 5
<211> 20
<212> DNA
<213> Artificial Sequence

<220>
<223> LYN forward primer

<400> 5
accagggagg agcccattta                                                    20

<210> 6
<211> 20
<212> DNA
<213> Artificial Sequence

<220>
<223> LYN reverse primer

<400> 6
cttccgctcg atgtatgcca                                                    20

<210> 7
<211> 24
<212> DNA
<213> Artificial Sequence

<220>
<223> PIK3CD forward primer

<400> 7
actctgccat tgtctaagcc acct                                               24

<210> 8
<211> 24
<212> DNA
<213> Artificial Sequence

<220>
<223> PIK3CD reverse primer

<400> 8
tcacagcagg ttcccaaagg tgat                                               24

<210> 9
<211> 22
<212> DNA
<213> Artificial Sequence

<220>
<223> PLCG2 forward primer

<400> 9
gcctgtccct ttgtagaagt gg                                                22

<210> 10
<211> 21
<212> DNA
<213> Artificial Sequence

<220>
<223> PLCG2 reverse primer

<400> 10
ggccattatc attcacaacc g                                                 21

<210> 11
<211> 21
<212> DNA
<213> Artificial Sequence

<220>
<223> BCL2 forward primer

<400> 11
ctgcacctga cgcccttcac c                                                 21

<210> 12
<211> 25
<212> DNA
<213> Artificial Sequence

<220>
<223> BCL2 reverse primer

<400> 12
cacatgaccc caccgaactc aaaga                                             25

<210> 13
<211> 18
<212> DNA
<213> Artificial Sequence

<220>
<223> BCL2L11 forward primer

<400> 13
ggcccctacc tccctaca                                                     18

<210> 14
<211> 21
<212> DNA
<213> Artificial Sequence

<220>

<223> BCL2L11 reverse primer

<400> 14
ggggtttgtg ttgatttgtc a                                                21


<210> 15
<211> 24
<212> DNA
<213> Artificial Sequence


<220>
<223> BID forward primer

<400> 15
cgcacctacg tgaggagctt agcc                                             24


<210> 16
<211> 26
<212> DNA
<213> Artificial Sequence


<220>
<223> BID reverse primer

<400> 16
tgaccacatc gagctttagc cagtca                                           26


<210> 17
<211> 26
<212> DNA
<213> Artificial Sequence


<220>
<223> BCL2L1 forward primer

<400> 17
gatccccatg gcagcagtaa agcaag                                           26


<210> 18
<211> 26
<212> DNA
<213> Artificial Sequence


<220>
<223> BCL2L1 reverse primer

<400> 18
ccccatcccg gaagagttca ttcact                                           26


<210> 19
<211> 20
<212> DNA
<213> Artificial Sequence

<220>
<223> PMAIP1 forward primer

<400> 19
cagagctgga agtcgagtgt                                                    20


<210> 20
<211> 20
<212> DNA
<213> Artificial Sequence


<220>
<223> PMAIP1 reverse primer

<400> 20
aggagtcccc tcatgcaagt                                                    20


<210> 21
<211> 21
<212> DNA
<213> Artificial Sequence


<220>
<223> CARD11 forward primer

<400> 21
ttgtgggaga atgtggagtg t                                                  21


<210> 22
<211> 21
<212> DNA
<213> Artificial Sequence


<220>
<223> CARD11 reverse primer

<400> 22
tgccccttgg tatgtagaat g                                                  21


<210> 23
<211> 25
<212> DNA
<213> Artificial Sequence


<220>
<223> B2M forward primer

<400> 23
tgctgtctcc atgtttgatg tatct                                              25


<210> 24
<211> 22
<212> DNA

<213> Artificial Sequence

<220>
<223> B2M reverse primer

<400> 24
tctctgctcc ccacctctaa gt                                                22

## Description

### Field of the invention

The present invention may be included in the field of medicine in general, more particularly in the field of pharmacology. In particular, the present invention is focused on the treatment of B-cell malignancies.

### Background to the invention

B-cell malignancies represent a diverse collection of diseases, including chronic lymphocytic leukaemia, follicular lymphoma, non-Hodgkin lymphoma, mantle cell lymphoma and diffuse large B-cell lymphoma. Of these, chronic lymphocytic leukemia (CLL), the most common leukemia in adults in western countries, is characterized by the progressive accumulation of mature-appearing clonal B cells expressing CD5, CD23 and CD19 surface markers in the blood, bone marrow and secondary lymphatic tissues.

While a subset of patients with CLL exhibit an indolent form of the disease, a progression of the malignancy is observed in subjects in which IgHV is not mutated, *NOTCH1* is mutated, chromosome 17p and/or chromosome 11q have been deleted. Response rates to currently available therapies in subjects with CLL remain highly variable, mostly due to intrinsic or acquired resistance to treatment and drug toxicity, as exemplified by fludarabine-induced lymphocytopenia due to T cell depletion, thus underscoring the need for treatments with a broader spectrum and safer effect independent of the cytogenetic profile of the subject.

The therapeutic armamentarium of subjects with B-cell malignancies has recently expanded toward agents that inhibit key processes such as B-cell receptor (BCR) signaling in CLL since its aberrant activation provides growth and survival signals to leukemia cells. Such agents include idelalisib, the phosphatidylinositol 3-kinase delta (PI3Kδ) inhibitor for treatment of B-cell malignancies. However, despite the clinical benefits demonstrated by novel agents such as this, a substantial fraction of patients eventually relapses.

It is therefore a problem of the present invention to provide means for treating a broad spectrum of B-cell malignancies such as CLL, wherein said means exhibits reduced toxicity and improved efficacy. It is a further problem of the present invention to provide means for treating a B-cell malignancy such as CLL which exhibits resistance to treatment.

### Brief description of the invention

The present invention relates to a compound of Formula (I), 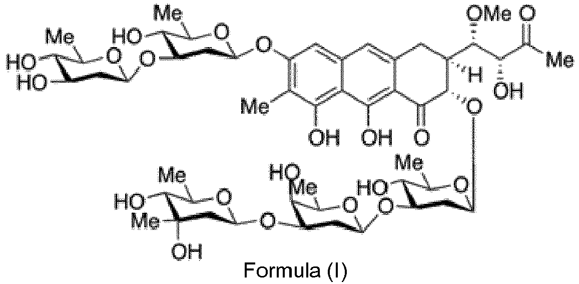 or a pharmaceutically acceptable salt, co-crystal or solvate thereof, for use in treatment of a B-cell malignancy, preferably chronic lymphocytic leukemia.

In addition, the present invention relates to a composition comprising:
(a) a compound of Formula (I), 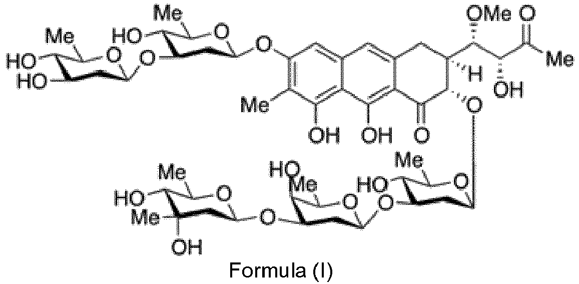 or a pharmaceutically acceptable salt, co-crystal or solvate thereof; and
(b) at least one chemotherapeutic agent selected from idelalisib or fludarabine, or a salt, co-crystal or solvate thereof.

Moreover, the present invention relates to the aforementioned composition for use as a medicament.

Additionally, the present invention relates to the aforementioned composition for use in treatment of chronic lymphocytic leukemia.

Furthermore, the present invention relates to a method for producing the aforementioned composition, which comprises mixing:
(i) a compound of Formula (I), 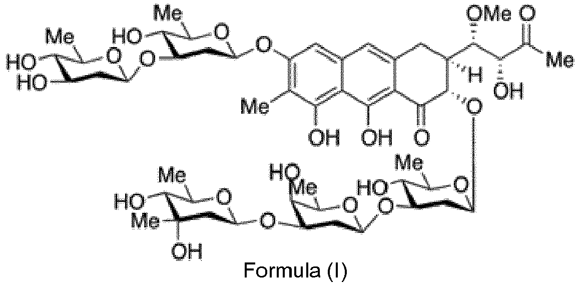 or a pharmaceutically acceptable salt, co-crystal or solvate thereof; and
(ii) at least one chemotherapeutic agent selected from idelalisib or fludarabine, or a salt, co-crystal or solvate thereof.

The present invention also relates to a kit-of-parts for use in treatment of chronic lymphocytic leukemia, comprising:
(a) a compound of Formula (I), 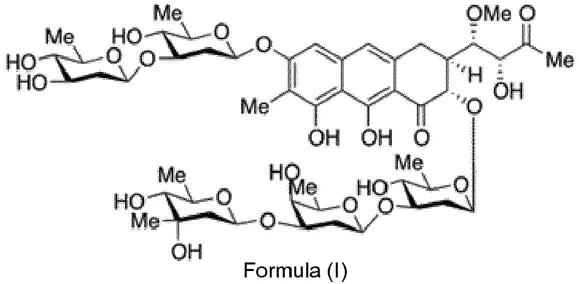 or a pharmaceutically acceptable salt, co-crystal or solvate thereof; and
(b) at least one chemotherapeutic agent selected from idelalisib or fludarabine, or a salt, co-crystal or solvate thereof.

The present invention further relates to a compound of Formula (I), 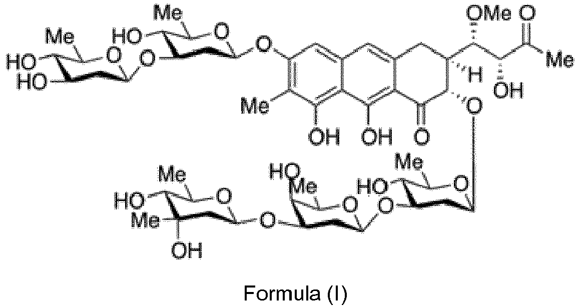 or a pharmaceutically acceptable salt, co-crystal or solvate thereof, for use in treatment of chronic lymphocytic leukaemia (CLL) in a human subject, independently of whether IgHV is mutated or not in said subject, and independently of whether chromosome 17p and/or chromosome 11q is deleted or not in said subject,
preferably wherein said compound of Formula (I), or a salt, co-crystal or solvate thereof, is administered simultaneously with, separately from or sequentially to the administration of at least one chemotherapeutic agent selected from idelalisib or fludarabine, or a salt, co-crystal or solvate thereof, wherein:
- when the chemotherapeutic agent is idelalisib, the molar ratio of the compound of the Formula (I) to idelalisib is from 1:3 to 1:300, and
- when the chemotherapeutic agent is fludarabine and the molar ratio of the compound of the Formula (I) to fludarabine is from 1:7 to 1:380.

The present invention also further relates to a composition comprising:
(a) a compound of Formula (I), 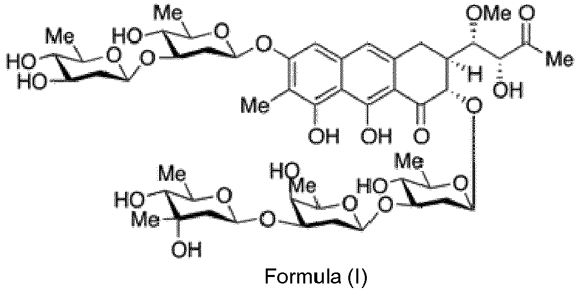 or a pharmaceutically acceptable salt, co-crystal or solvate thereof; and
(b) at least one chemotherapeutic agent selected from idelalisib or fludarabine, or a salt, co-crystal or solvate thereof,
wherein:
- when the chemotherapeutic agent is idelalisib, the molar ratio of the compound of the Formula (I) to idelalisib is from 1:3 to 1:300, and
- when the chemotherapeutic agent is fludarabine and the molar ratio of the compound of the Formula (I) to fludarabine is from 1:7 to 1:380,
preferably wherein said composition is for use in treatment of chronic lymphocytic leukaemia (CLL) in a human subject, independently of whether IgHV is mutated or not in said subject, and independently of whether chromosome 17p and/or chromosome 11q is deleted or not in said subject.

### Description of the figures

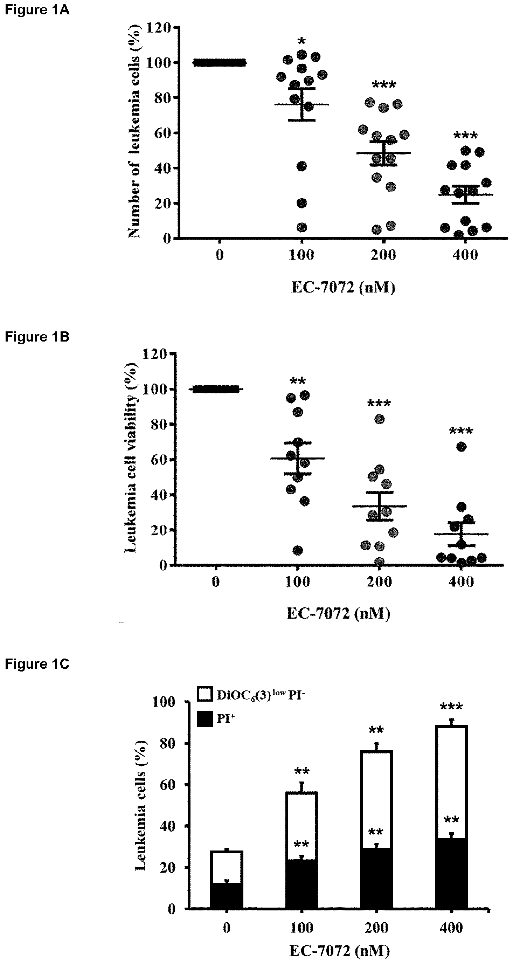

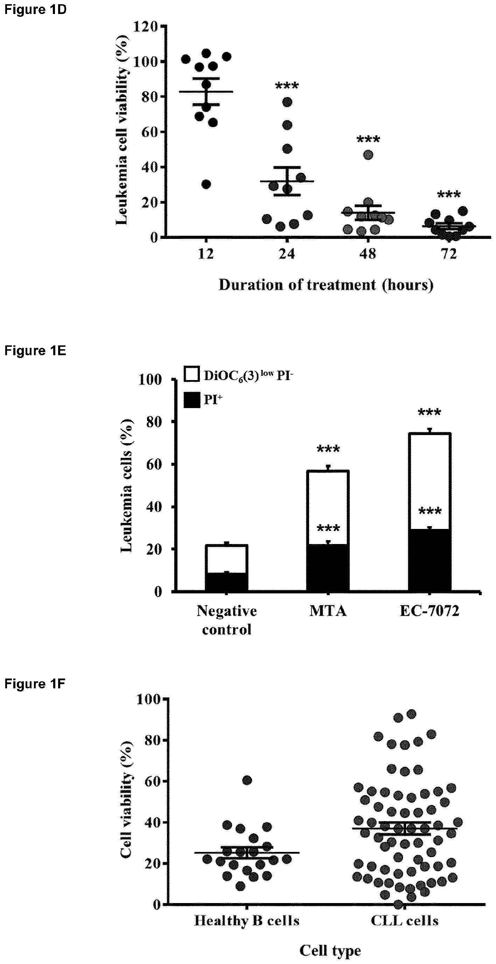

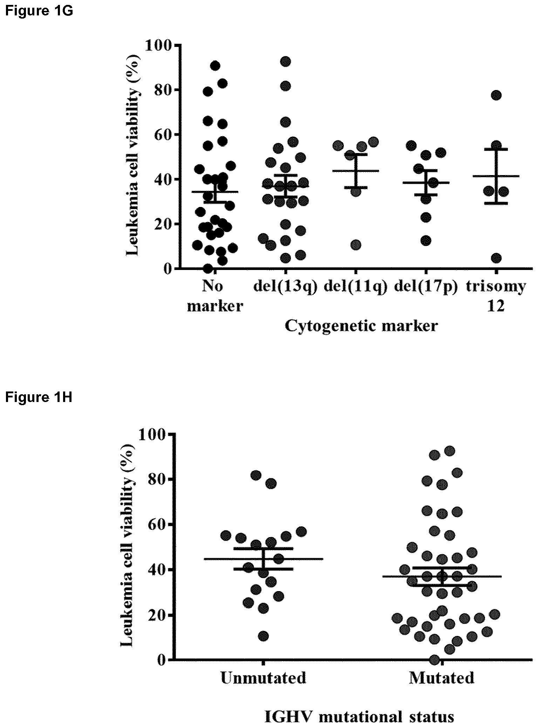

**Figure 1****.** Graphs representing: **A.** the number of cells normalized to their respective control (DMSO) condition for each individual patient following incubation of PBMCs from patients with CLL (n = 13) when treated with increasing concentrations of EC-7072 (0 - 400 nM) for 24 hours, whereby numbers of leukemia cells were evaluated by flow cytometry; **B.** the percentage viability of leukemia cells following incubation of PBMCs from patients with CLL (n = 10) with or without EC-7072 (100 - 400 nM) for 24 hours, whereby viability of leukaemia cells was determined by cytofluorometric assessment of DiOC₆(3)/PI staining; **C**. the mean percentages of apoptotic [DiOC₆(3)^{low} PI⁻] and dead [PI⁺] cells following incubation of peripheral blood mononuclear cells (PBMCs) from patients with CLL (n = 10) when treated with increasing doses (0 to 400 nM) of EC-7072 for 24 hours, whereby apoptosis of leukemia cells was determined by DiOC₆(3)/PI staining and flow cytometry; **D**. the percentage viability of leukemia cells following incubation of PBMCs from patients with CLL (n = 10) with or without EC-7072 (200 nM) for 12 to 72 hours, whereby viability of leukaemia cells was determined by cytofluorometric assessment of DiOC₆(3)/PI staining; **E**. the mean percentages of apoptotic [DiOC₆(3)^{low} PI⁻] and dead [PI⁺] cells following incubation of peripheral blood mononuclear cells (PBMCs) from patients with CLL (n = 24) when treated with MTA (200 nM) or EC-7072 (200 nM)for 24 hours, whereby leukemia cell death was evaluated by DiOC₆(3)/PI staining. Bars represent the mean percentages of apoptotic [DiOC₆(3)^{low} PI⁻] and dead [PI⁺] cells (mean ± SEM) (**P < 0.01; ***P < 0.001; Student's t-test); **F**. the percentage of viable [DiOC₆(3)⁺] cells normalized to their respective control condition following incubation of PBMCs from patients with CLL (n = 63) or healthy donors (n = 20) with or without EC-7072 (200 nM) for 24 hours, whereby viability of leukemia cells and healthy B cells was evaluated by DiOC₆(3)/PI staining; **G.** the percentage viability of leukemia cells following incubation of PBMCs from patients with CLL (n = 62) when treated with EC-7072 (200 nM) for 24 hours, whereby the cytogenetic profile was obtained for each patient and viability of leukemia cells was evaluated by DiOC₆(3)/PI staining; and **H.** the percentage of viable [DiOC₆(3)⁺] cells normalized to their respective control (DMSO) condition for each individual patient, following incubation of PBMCs from patients with CLL (n = 59) when treated with EC-7072 (200 nM) for 24 hours, whereby mutational status of IgHV was determined for each patient and viability of leukemia cells was evaluated by DiOC₆(3)/PI staining. Dark lines in **Figures 1A, 1B****,** **1D** and **1F** to **1H** represent mean ± standard error of the mean (SEM) (*P < 0.05; **P < 0.01; ***P < 0.001; Student's t-test). Bars in **Figures 1A** and **1E**, represent the mean percentages of apoptotic [DiOC₆(3)^{low} PI⁻] and dead [PI⁺] cells. (Mean ± SEM) (**P < 0.01; ***P < 0.001; Student's t-test).

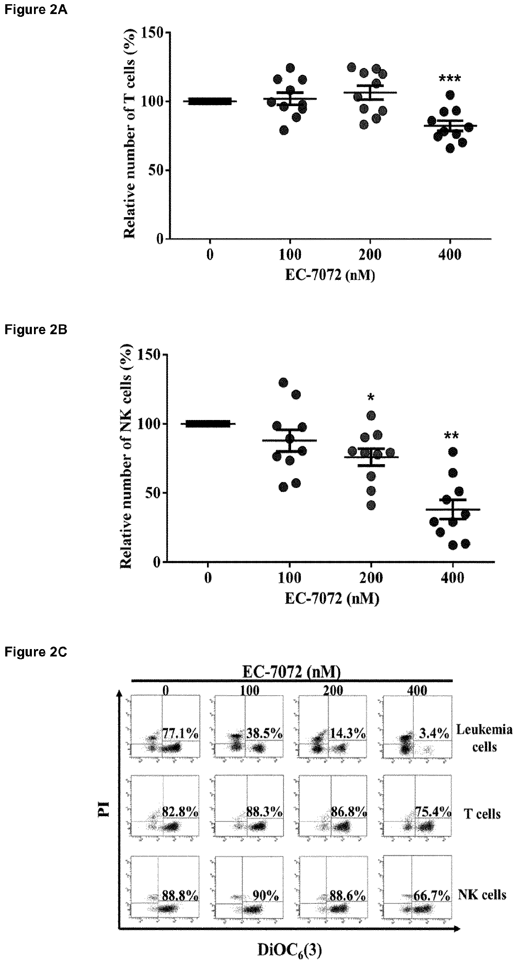

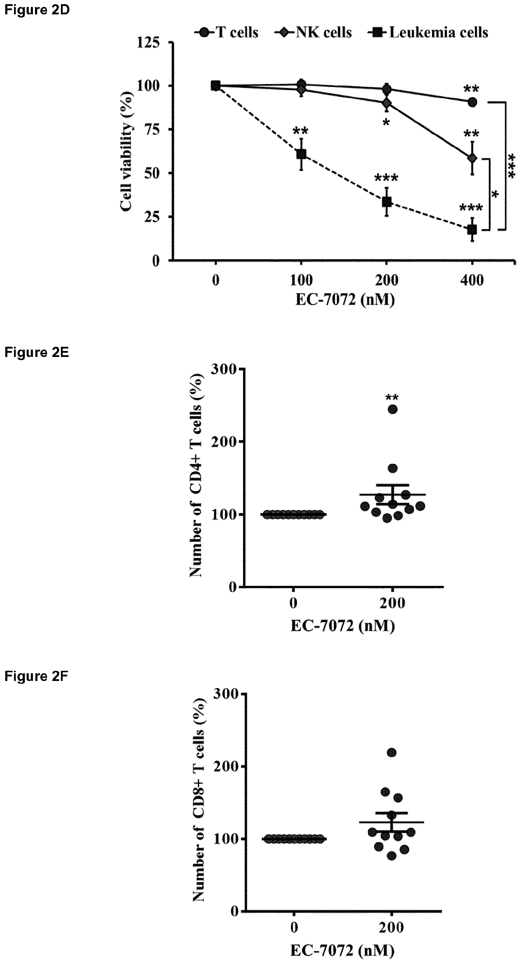

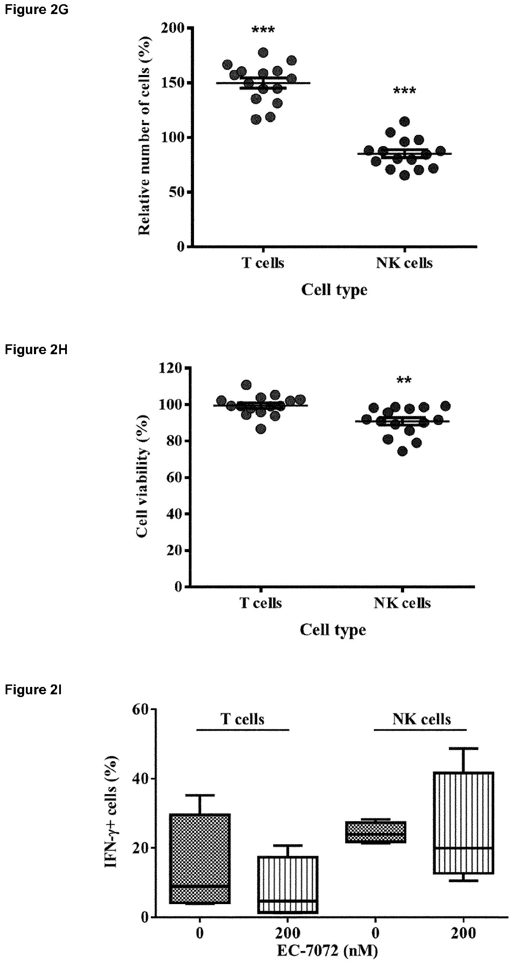

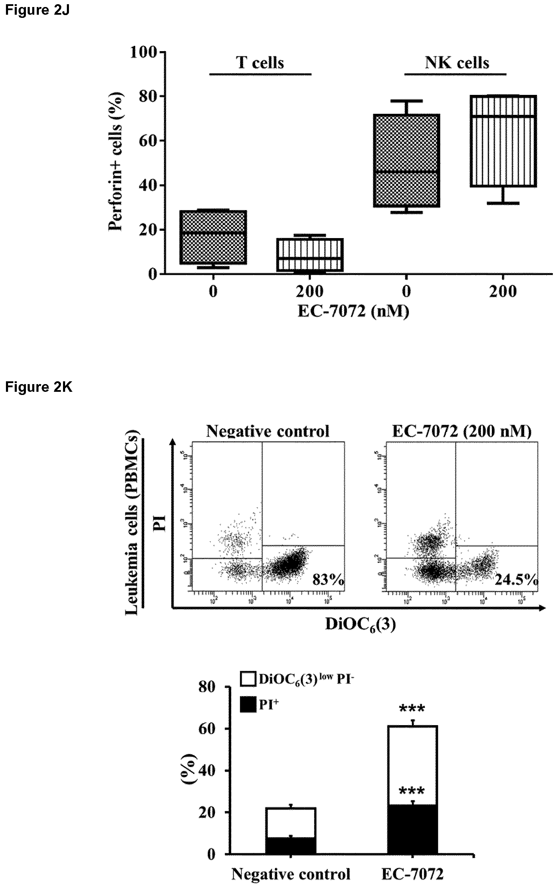

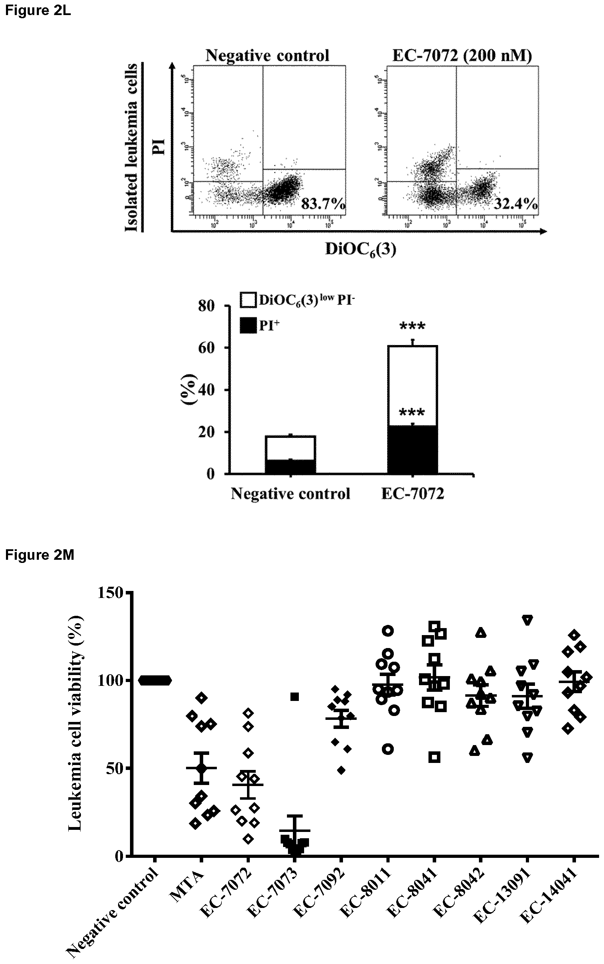

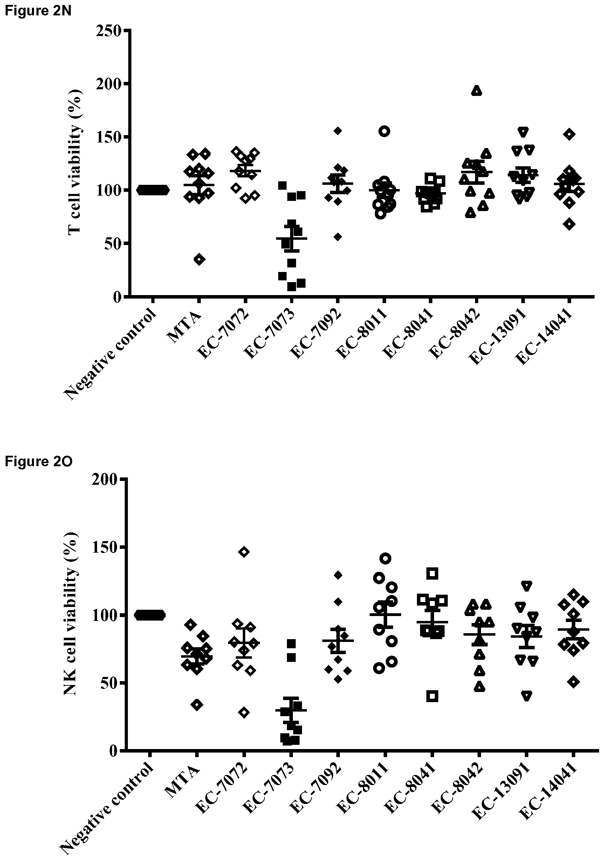

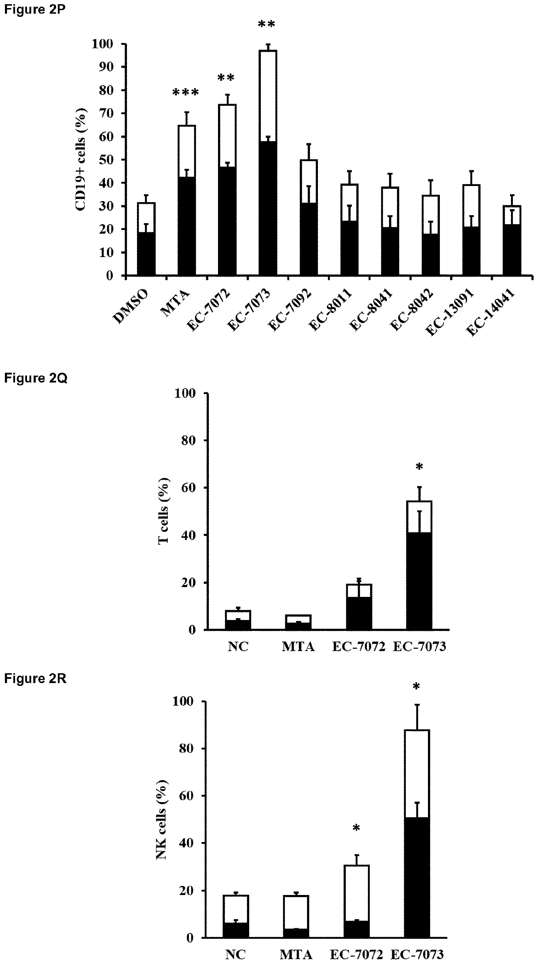

**Figure 2****.** Graphs representing: **A.** the number of T cells (CD3⁺CD56⁻) and **B.** the number of NK cells (CD3⁻CD56⁺), each normalized to their respective control (DMSO) condition for each individual patient, following incubation of PBMCs from patients with CLL (n = 10) with increasing concentrations of EC-7072 (0 - 400 nM) for 24 hours, whereby numbers of cells were evaluated by flow cytometry and dark lines correspond to mean ± SEM; **C.** dot plots depicting a representative patient with CLL whereby percentages within refer to viable [DiOC₆(3)⁺] cells and **D.** the percentage of viable [DiOC₆(3)⁺] cells normalized to their respective control (DMSO) condition, each obtained following incubation of PBMCs from patients with CLL (n = 6 - 10) with increasing concentrations of EC-7072 (0 - 400 nM) for 24 hours whereby viability of immune subsets (leukemia cells, T cells and NK cells) was determined by cytofluorometric assessment of DiOC₆(3)/PI staining; **E.** the number of CD4⁺ T cells and **F.** the number of CD8⁺ T cells, each normalized to their respective control (DMSO) condition for each individual experiment following incubation of PBMCs from patients with CLL (n = 11) DMSO or EC-7072 (200 nM) for 24 hours, whereby the numbers of T lymphocytes were evaluated by flow cytometry and dark lines correspond to mean ± SEM (**P < 0.01; Student's t-test); **G.** percentage of IFN-γ⁺ and **H.** percentage of perforin⁺ cells, each obtained following incubation of PBMCs from patients with CLL (n = 4) exposed to EC-7072 (200 nM) for 24 hours, whereby IFN-γ and perforin expression upon PMA/ionomycin stimulation was evaluated in T cells and NK cells by intracellular flow cytometry; **I.** dot plots depicting a representative patient whereby percentages within refer to viable [DiOC₆(3)⁺] cells, and **J.** the percentages of apoptotic [DiOC₆(3)^{low} PI⁻] and dead [PI⁺] cells (mean ± SEM) (*P < 0.05; **P < 0.01; ***P < 0.001; Student's t-test), each obtained following incubation of PBMCs from patients with CLL (n = 24) treated with EC-7072 (200 nM) for 24 hours, whereby apoptosis was examined by DiOC₆(3)/PI staining; dot plots (upper panels) depicting a representative patient whereby percentages within refer to viable [DiOC₆(3)⁺] cells, and the percentages of apoptotic [DiOC₆(3)^{low} PI⁻] and dead [PI⁺] cells (mean ± SEM) (*P < 0.05; **P < 0.01; ***P < 0.001; Student's t-test) (lower panels), each obtained following incubation of **L.** PMBCs or **K.** isolated leukemia cells from patients with CLL (n = 24) treated with EC-7072 (200 nM) for 24 hours, whereby apoptosis was examined by DiOC₆(3)/PI staining; **M.** percentage viability of leukemia cells obtained following incubation of PBMCs isolated from patients with CLL (n = 10) following treatment with MTA (200 nM), EC-7072 (200 nM) or other mithralogs (EC-7073, EC-7092, EC-8011, EC-8041, EC-8042, EC-13091 or EC-14041, each at 200 nM) for 24 hours, whereby viability of leukemia cells was evaluated by flow cytometry and normalized to the respective control (DMSO) condition for each individual experiment; percentage viability of **N.** T cells and **O.** NK cells obtained following incubation of PBMCs isolated from healthy donors (n = 10) after treatment with MTA (200 nM), EC-7072 (200 nM) or other mithralogs (EC-7073, EC-7092, EC-8011, EC-8041, EC-8042, EC-13091 or EC-14041, each at 200 nM) for 24 hours, whereby viability of T cells and NK cells was evaluated by flow cytometry and normalized to the respective control (DMSO) condition for each individual experiment; **P.** mean percentages of apoptotic [DiOC₆(3)^{low} PI⁻] and dead [PI⁺] CD19⁺ cells obtained following incubation of PBMCs from patients with CLL (n = 6) with MTA (200 nM), EC-7072 (200 nM) or other mithralogs (EC-7073, EC-7092, EC-8011, EC-8041, EC-8042, EC-13091 or EC-14041, each at 200 nM) for 24 hours, whereby leukemia cell death was determined by DiOC₆(3)/PI staining and numbers of CD19⁺ cells were evaluated by flow cytometry; mean percentages of apoptotic [DiOC₆(3)^{low} PI⁻] and dead [PI⁺] **Q.** T cells and **R.** NK cells obtained following incubation of PBMCs isolated from healthy donors (n = 4) following treatment with MTA (200 nM), EC-7072 (200 nM) or EC-7073 (200 nM) for 24 hours, whereby leukemia cell death was determined by DiOC₆(3)/PI staining and numbers of T cells and NK cells were evaluated by flow cytometry (Mean ± SEM; **P* < 0.05; ***P* < 0.01; ****P* < 0.001; Student's *t*-test).

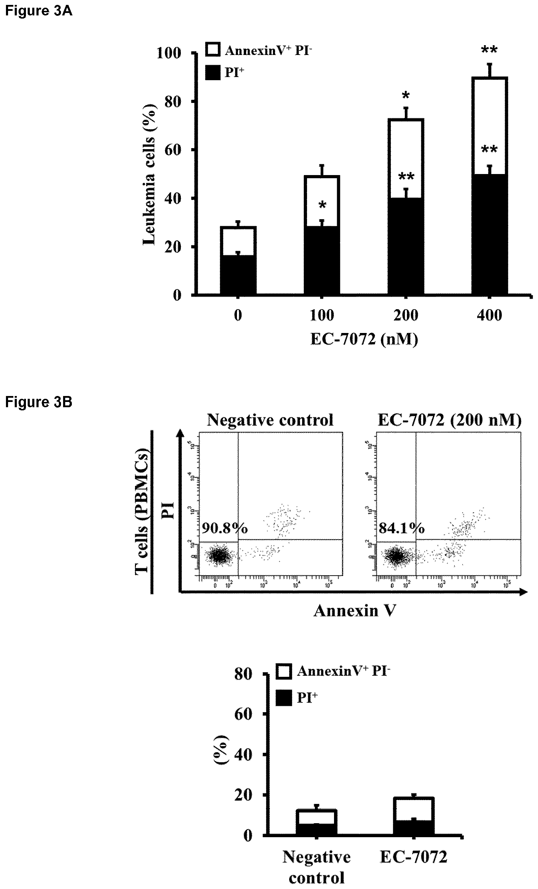

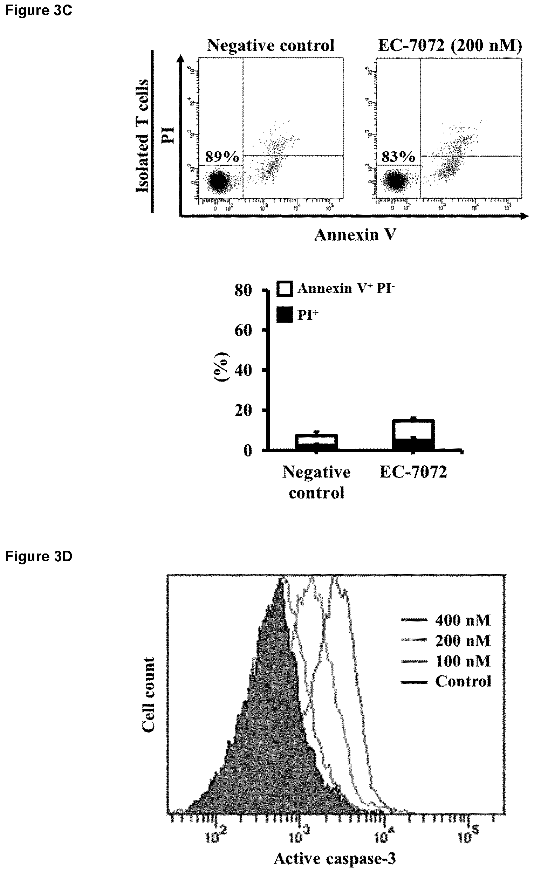

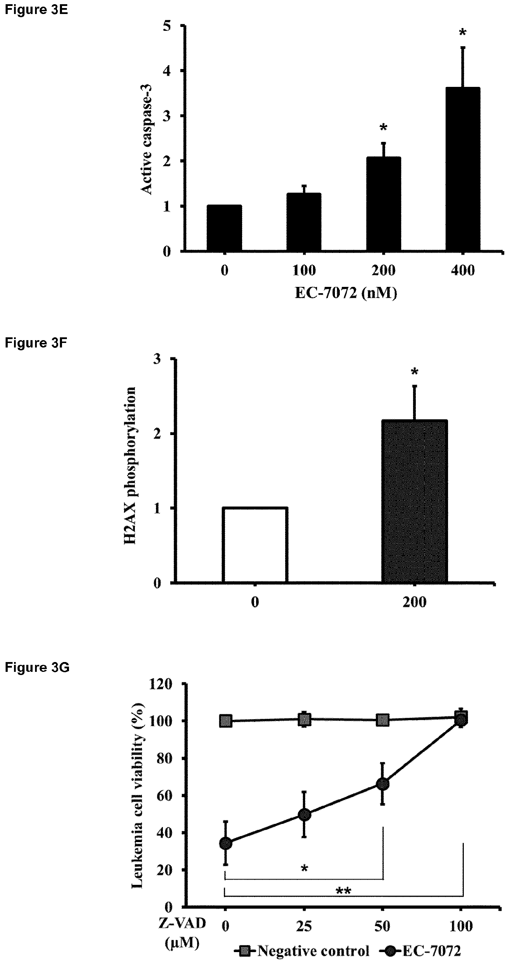

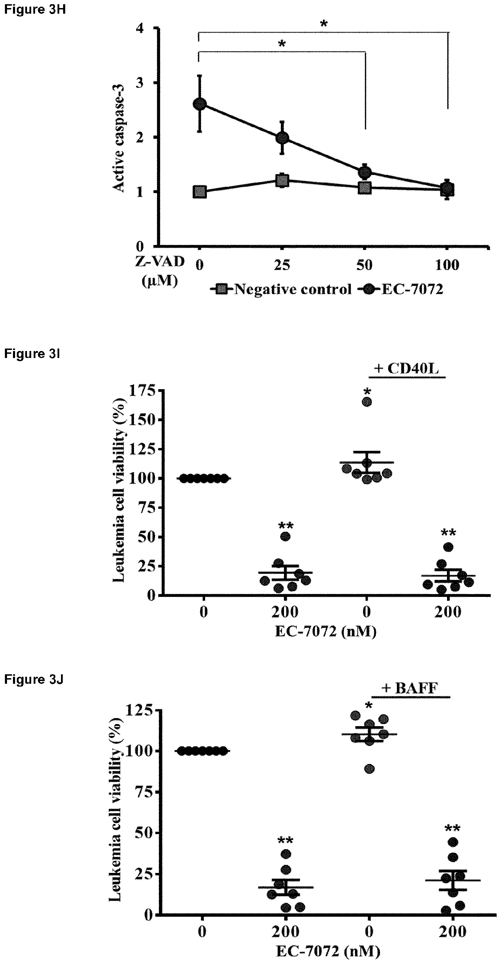

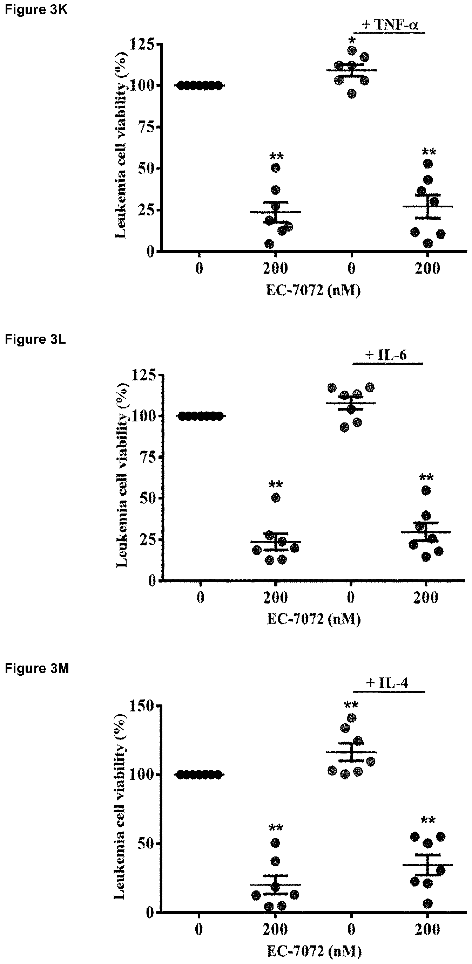

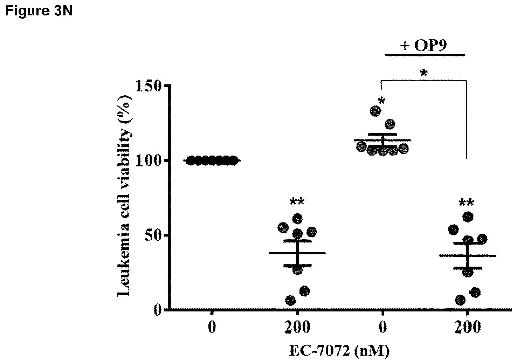

**Figure 3**. Graphs representing: **A.** the percentages of apoptotic [Annexin V⁺ PI⁻] and dead [PI⁺] cells following incubation of PBMCs from patients with CLL (n = 6) treated with EC-7072 (0 - 400 nM) for 24 hours, whereby apoptosis of leukemia cells was evaluated by cytofluorometric assessment of Annexin V/PI staining; **B.** dot plots showing a representative patient wherein percentages within refer to viable [Annexin V⁻] cells and **C.** the mean percentages of apoptotic [Annexin V⁺ PI⁻] and dead [PI⁺] cells (mean ± SEM) (Student's *t*-test), each obtained following incubation of PBMCs and isolated T cells from patients with CLL (n = 3) treated with EC-7072 (200 nM) for 24 hours, whereby T cell apoptosis was examined by cytofluorometric assessment of Annexin V/PI staining; **D.** histograms illustrating the MFI of a representative patient and **E.** the MFI normalized to the control (DMSO) condition, each obtained following incubation of PBMCs from patients with CLL (n = 6) treated with EC-7072 (0 - 400 nM) for 24 hour, whereby levels of active caspase-3 in leukemia cells were assessed by flow cytometry; **F.** the MFI normalized to the control (DMSO) condition following incubation of isolated leukemia cells from patients with CLL (n = 4) treated with EC-7072 (200 nM) for 12 hours, whereby levels of phosphorylated H2AX were assessed by phosphoflow; **G.** the percentage of viable [DiOC₆(3)⁺] cells and **H.** the MFI, each normalized to the control (DMSO) condition following incubation of PBMCs from patients with CLL (n = 6) pretreated with increasing doses of Z-VAD-fmk (0-100 µM) followed by exposure to EC-7072 (200 nM) for 24 hours, whereby viability of leukemia cells was evaluated by DiOC₆(3)/PI staining and levels of active caspase-3 were examined by flow cytometry in leukemia cells; the mean percentage of viable [DiOC₆(3)⁺] cells normalized to their respective control (DMSO) condition for each individual experiment following incubation of PBMCs from patients with CLL (n = 7) with 200 nM EC-7072 in combination with I. CD40L (100 ng/mL), **J.** BAFF (50 ng/mL), **K.** TNF-α (40 ng/mL), **L.** IL-6 (40 ng/mL) and **M.** IL-4 (40 ng/mL) for 24 hours, whereby leukemia cell viability was assessed by DiOC₆(3)/PI staining; and **N.** the percentage of viable [DiOC6(3)⁺] cells normalized to their respective control (DMSO) condition for each individual patient following incubation of PBMCs from patients with CLL (n = 7) that were co-cultured with OP9 cells for 72 hours and were exposed to 200 nM EC-7072 for the last 24 hours, whereby viability of leukemia cells was evaluated by DiOC₆(3)/PI staining. (Mean ± SEM) (**P* < 0.05; ***P* < 0.01; Student's *t*-test).

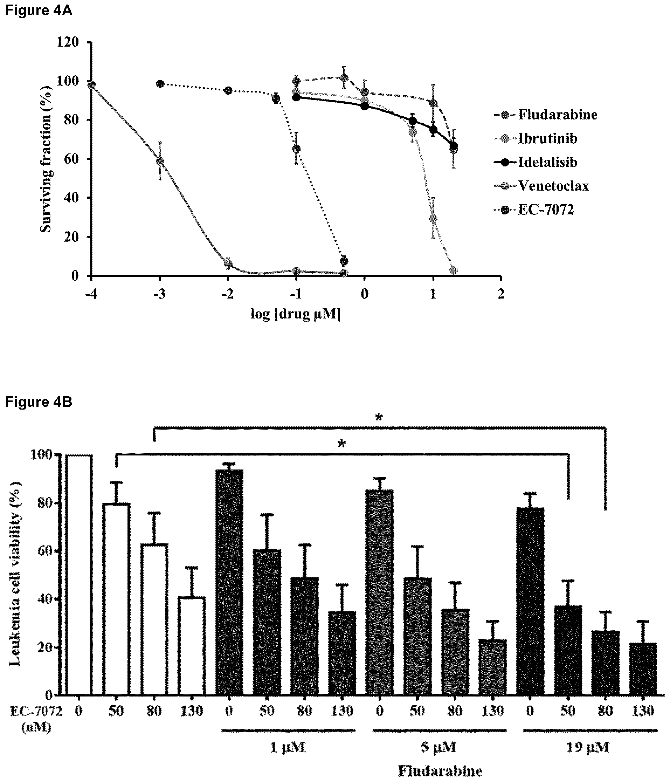

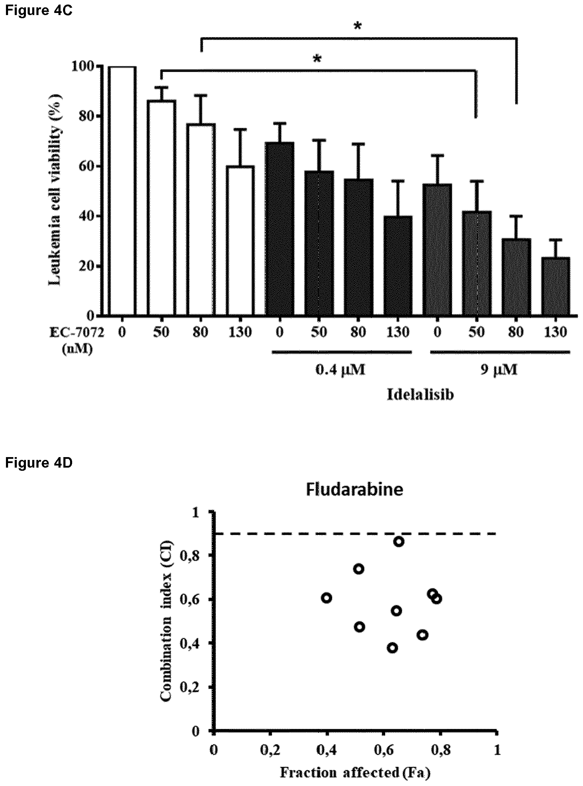

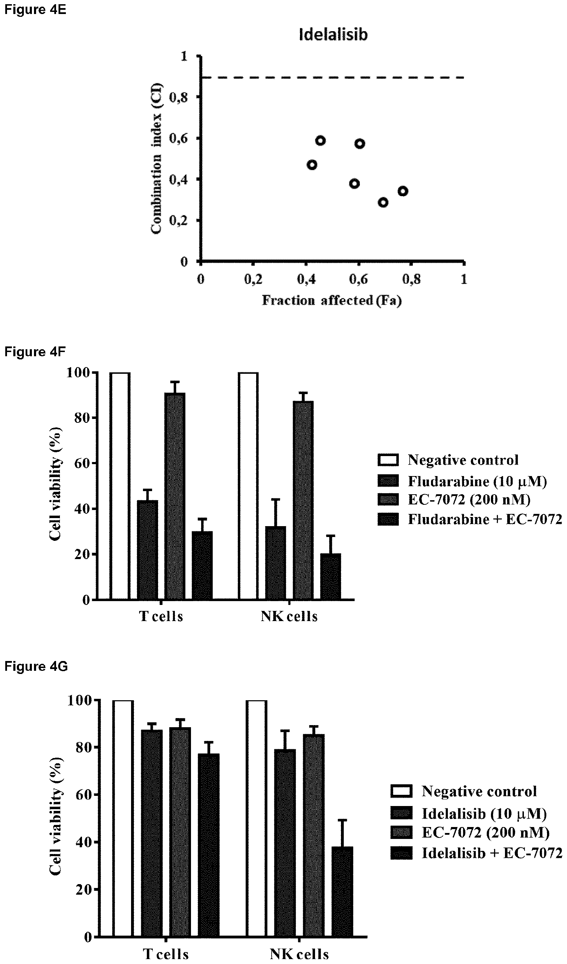

**Figure 4****.** Graphs representing **A.** the percentage of viable [DiOC₆(3)⁺] cells normalized to their respective control (DMSO) condition following incubation of PBMCs from patients with CLL (n = 4 - 6) with increasing concentrations of fludarabine, venetoclax or EC-7072 for 24 hours or ibrutinib or idelalisib for 48 hours, whereby CLL cell viability was assessed by DiOC₆(3)/PI staining; the percentage of viable [DiOC₆(3)⁺] cells normalized to their respective control (DMSO) condition (mean ± SEM) (**P* < 0.05; ***P* < 0.01; One-way ANOVA) following incubation of primary leukemia cells from patients with CLL (n = 6) upon exposure to non-fixed ratio combinations of EC-7072 with **B.** fludarabine and **C.** idelalisib, whereby concentrations employed correspond to IC₁₀, IC₂₅ and IC₅₀ values for fludarabine and IC₁₀, and IC₂₅ values for idelalisib, and leukemia cell viability was assessed by DiOC₆(3)/PI staining; CI plots generated for non-fixed ratio combinations of **D.** EC-7072 and fludarabine and **E.** EC-7072 and idelalisib, each using CalcuSyn software according to the Chou-Talalay method, whereby CI values < 0.9 indicate synergism, values between 0.9 - 1.1 correspond to additive effects and values > 1.1 represent antagonism, and the fraction affected (Fa) was calculated based on the percentage of viable cells after the treatment; and the percentage of viable [DiOC₆(3)⁺] cells normalized to their respective control (DMSO) condition in healthy immune subsets (T cells and NK cells) following incubation of PBMCs from patients with CLL (n = 6 - 8) after treatment with **F.** fludarabine (10 µM) for 24 hours and **G.** idelalisib (10 µM) for 48 hours, wherein EC-7072 (200 nM) was added to the cell culture for the last 24 hours of treatment, and whereby CLL cell viability was assessed by DiOC₆(3)/PI staining. Asterisks lacking a line underneath correspond to the significance vs the untreated control. (Mean ± SEM) (**P* < 0.05; ***P* < 0.01; ****P* < 0.001; One-way ANOVA).

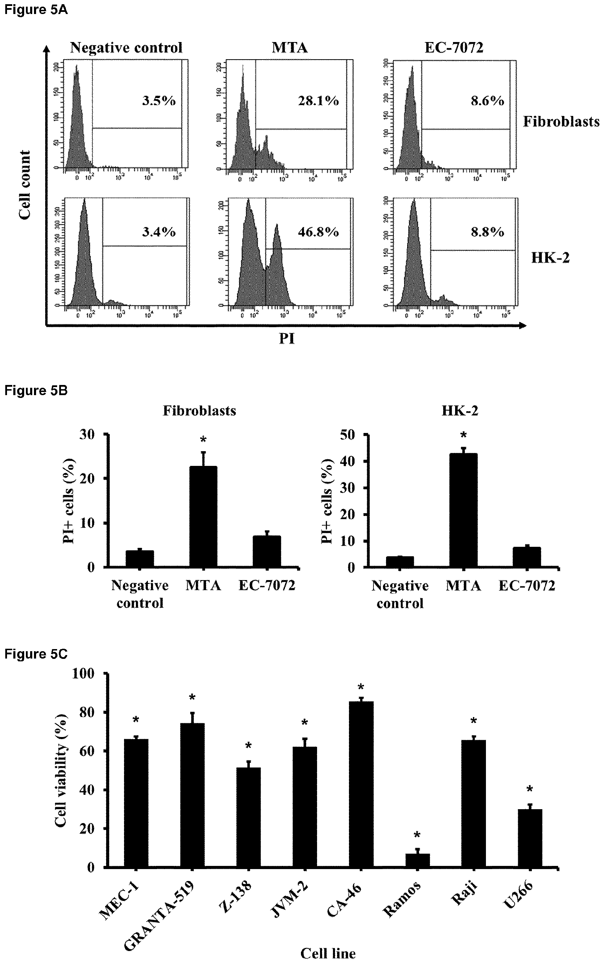

**Figure 5****.** Primary fibroblasts from healthy donors (n = 4) and HK-2 cells from healthy human adult kidney (n = 3) were treated with MTA (200 nM) or EC-7072 (200 nM) for 24 hours and cell death was evaluated by PI staining, resulting in **A.** histograms depicting a representative experiment wherein percentages within correspond to the PI⁺ subset and **B.** bar graphs depicting bars representing the mean percentage of PI⁺ cells (mean ± SEM) (**P* < 0.05; Student's *t*-test); and **C.** bar graphs representing the mean percentages of viable [DiOC₆(3)⁺] cells normalized to control (DMSO) condition (mean ± SEM) (**P* < 0.05; Student's *t*-test) following incubation of the B-cell malignancy cell lines indicated therein when treated with EC-7072 (500 nM) for 24 hours, whereby cell viability was assessed by DiOC₆(3)/PI staining (n = 3).

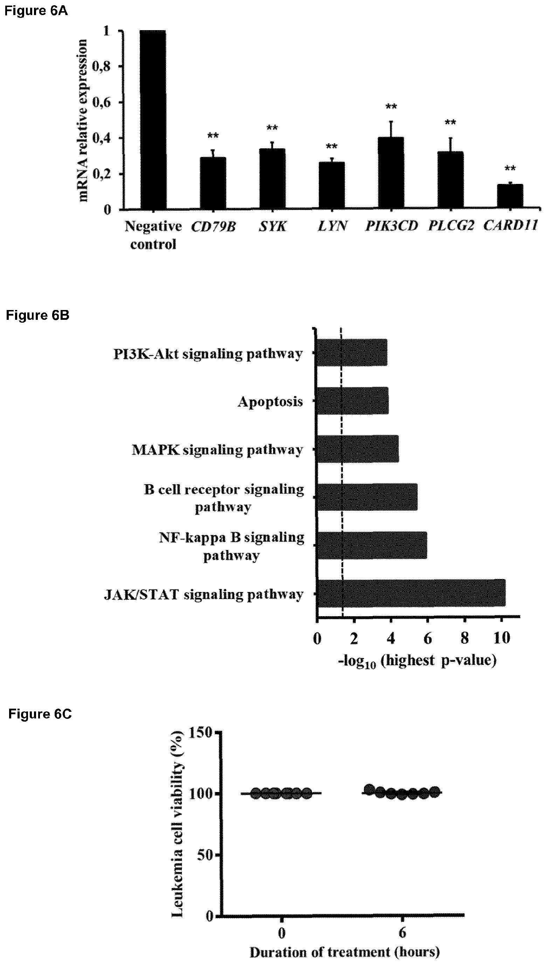

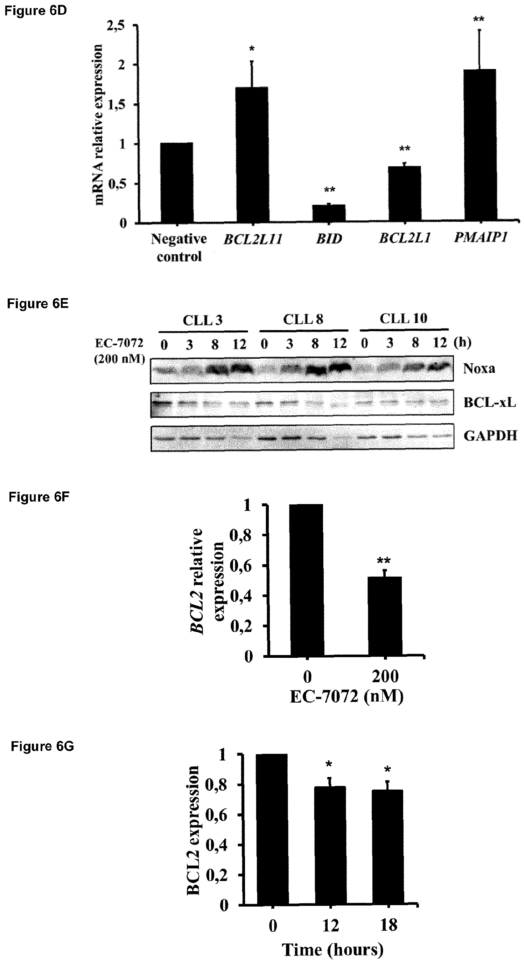

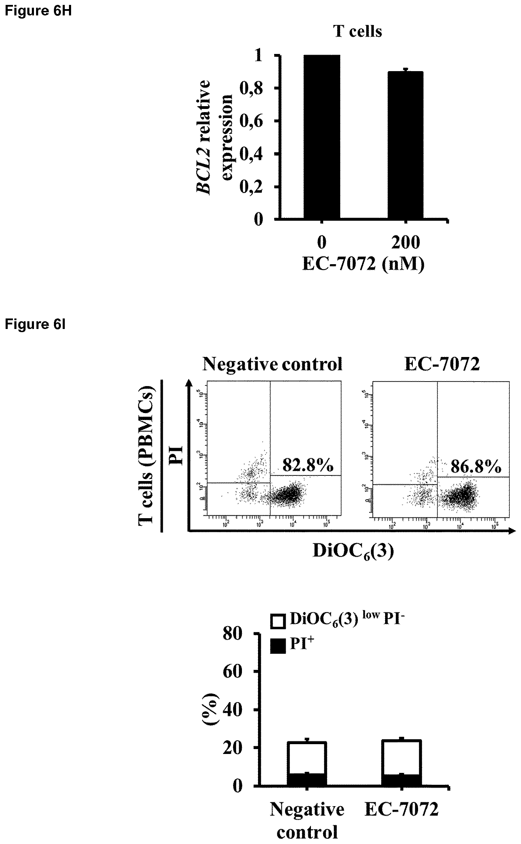

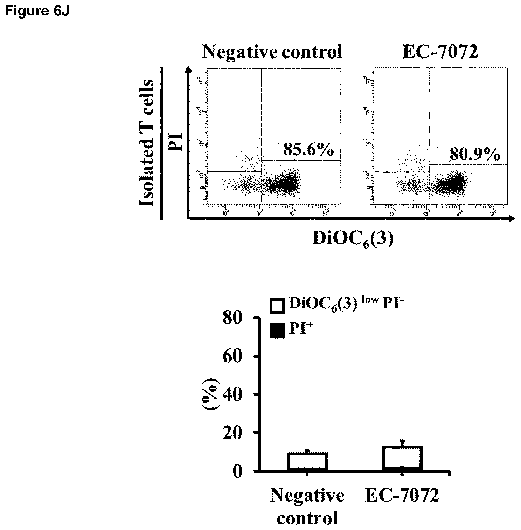

**Figure 6****.** Graphs depicting **A.** bars representing the mean relative expression normalized to the control (DMSO) condition following analysis of the expression of BCR-related genes by qPCR in isolated CLL leukemia cells (n = 6) exposed to 200 nM EC-7072 for 6 hours (mean ± SEM); **B.** bars representing the -log₁₀ of the highest p-value obtained from the iterative pathway analysis, whereby selected relevant pathways for CLL cell homeostasis were significantly modulated by EC-7072 obtained through KEGG pathway analysis (dashed line corresponds to p-value = 0.05); C. the mean percentage of viable [DiOC₆(3)⁺] cells normalized to the control (DMSO) condition for each individual patient following incubation of PBMCs from patients with CLL (n = 8), after treatment with 200 nM EC-7072, for 6 hours, whereby viability was assessed by DiOC₆(3)/PI staining and the dark lines represent mean ± SEM; **D.** bars representing the mean relative expression to the control (DMSO) control condition (mean ± SEM), following analysis of expression of relevant genes that regulate apoptosis in CLL by qPCR in isolated leukemia cells from patients with CLL (n = 6) exposed to 200 nM EC-7072 for 6 hours; **E.** Western blot analysis of BCL-xL and Noxa in isolated CLL cells (n = 3) treated with 200 nM EC-7072 for the indicated times; **F.** bars depicting the mean relative expression to the control (DMSO) condition (mean ± SEM, following incubation of isolated CLL cells (n = 6) from patients with CLL after treatment with EC-7072 (200 nM) for 6 hours, whereby total RNA was extracted and relative expression of *BLC2* was determined by qPCR; **G.** BCL2 levels evaluated by intracellular flow cytometry (mean ± SEM) following incubation of isolated CLL cells (n = 6) after treatment with 200 nM EC-7072 for 12 and 18 hours; **H.** bars depicting the mean relative expression to the control (DMSO) condition (mean ± SEM) following incubation of isolated T cells (n = 3) from patients with CLL after treatment with EC-7072 (200 nM) for 6 hours, whereby total RNA was extracted and relative expression of *BLC2* was determined by qPCR; and dot plots showing a representative patient and percentages within referring to viable [DiOC₆(3)⁺] cells, and graphs depicting the mean percentages of apoptotic [DiOC₆(3)^{low} PI⁻] and dead [PI⁺] cells (mean ± SEM) following incubation of **I.** PBMCs and **J.** isolated T cells from patients with CLL (n = 3) after treatment with 200 nM EC-7072 for 24 hours, whereby T-cell death and apoptosis were assessed by DiOC₆(3)/PI staining. (**P* < 0.05; ***P* < 0.01; Student's *t*-test).

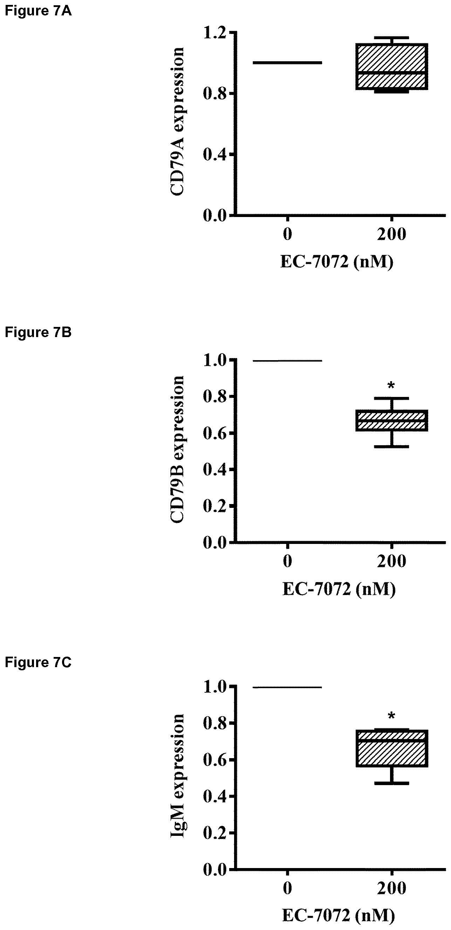

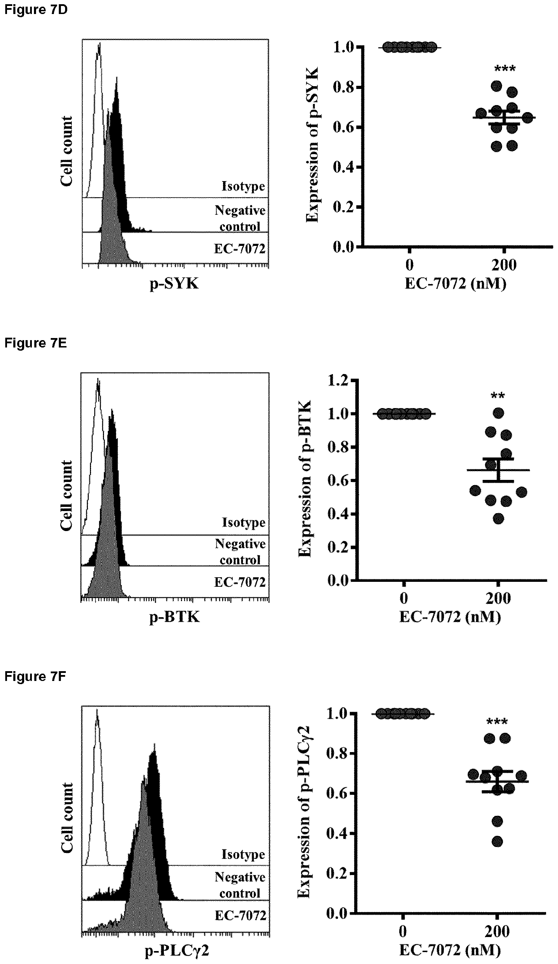

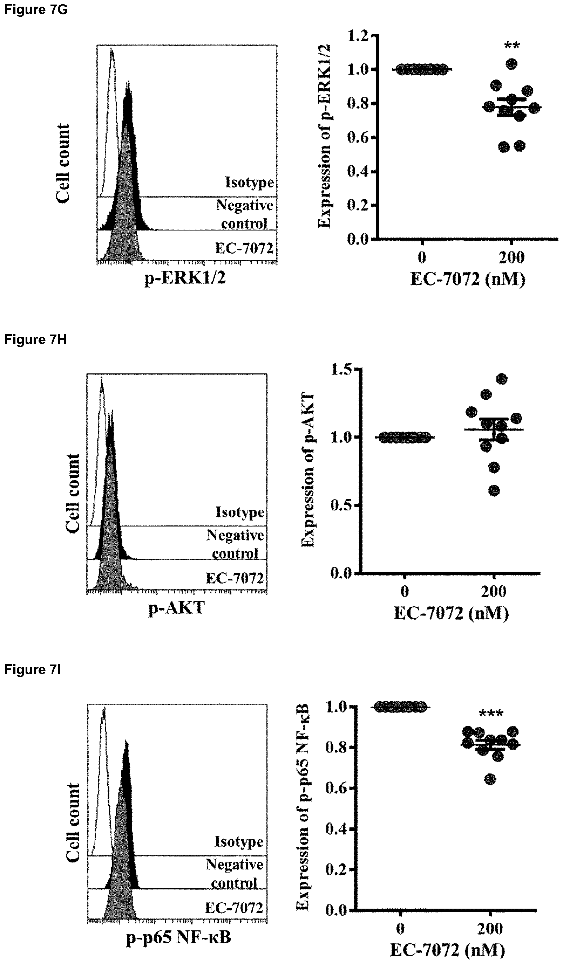

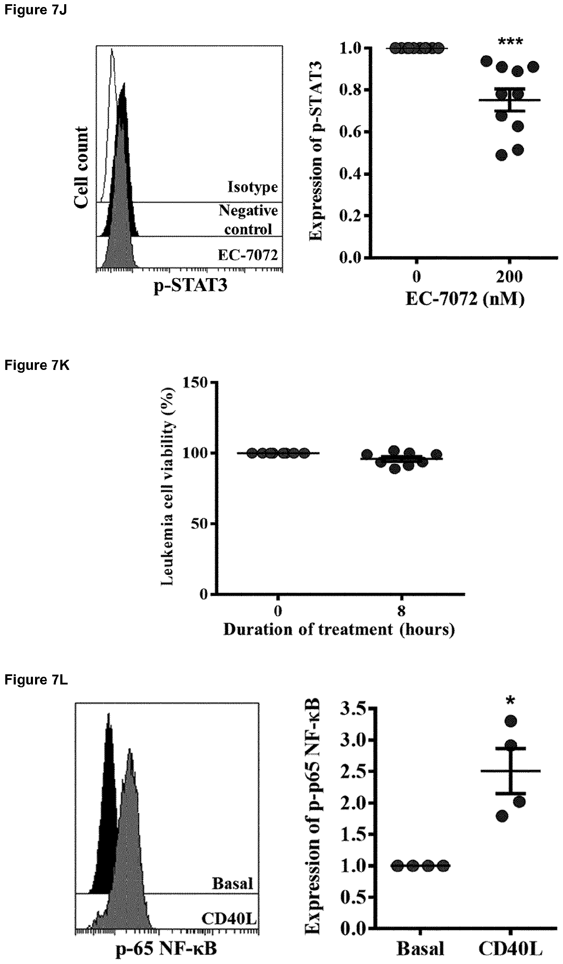

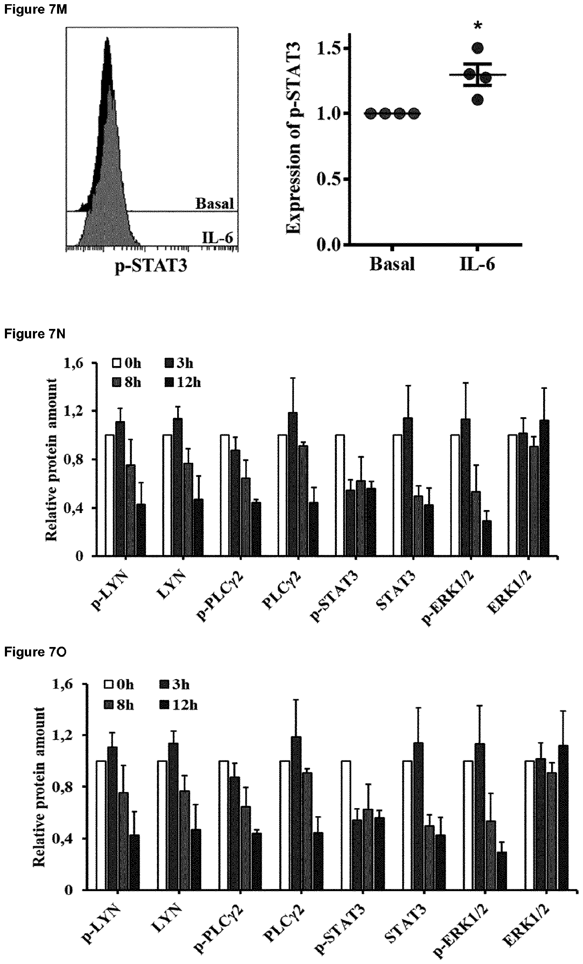

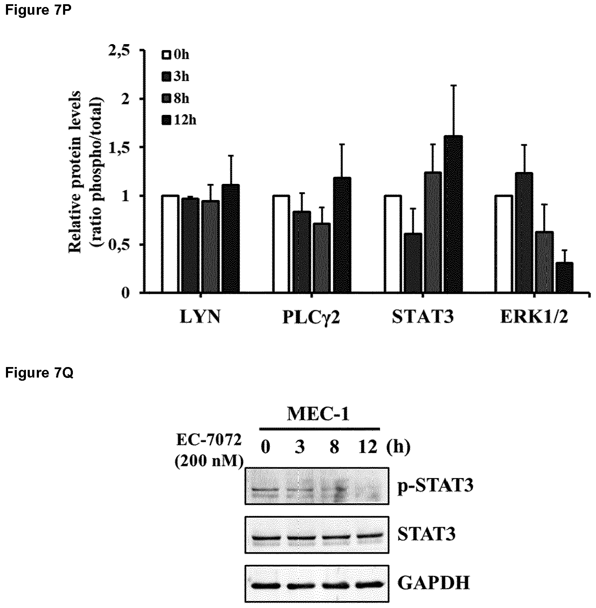

**Figure 7****.** Graphs representing the surface expression of A. CD79A, **B.** CD79B and C. IgM in leukemia cells (n = 5-6) following assessment by flow cytometry MFI normalized to the control (DMSO) condition after PBMCs from patients with CLL were treated with 200 nM EC-7072 for 8 hours, whereby boxes represent the MFI normalized to the control (DMSO) condition (**P* < 0.05; Student's *t*-test); histograms depicting the MFI from a representative patient ((upper panels) and graphs represent the MFI normalized to the control (DMSO) from each individual patient (lower panels) of the phosphorylation levels for the **D.** p-SYK, **E.** p-BTK, **F.** p-PLCγ, **G.** p-ERK1/2, **H.** p-AKT, **I.** p-p65 NPκB and **J.** p-STAT3 proteins, whereby phosphorylation levels were determined by phosphoflow in isolated leukemia cells from patients with CLL (n = 10) incubated with EC-7072 (200 nM) for 8 hours, wherein dark lines correspond to mean ± SEM (B and D) (***P* < 0.01; ****P* < 0.001; Student's *t*-test); **K**. graph depicting the mean percentage of viable [DiOC₆(3)⁺] cells normalized to the control (DMSO) condition for each individual patient following incubation of PBMCs from patients with CLL (n = 8), after treatment with EC-7072 (200 nM), for 8 hours, whereby viability was determined by DiOC₆(3)/PI staining and dark lines represent mean ± SEM; graphs representing the MFI of a representative patient and the mean MFI normalized to the control (DMSO) condition for each individual patient, whereby phosphorylation levels of **L.** p65 NF-κB and **M.** STAT3 were detected by phosphoflow in isolated CLL cells (n = 4) stimulated with CD40L (100 ng/mL) or IL-6 (40 ng/mL) for 15 minutes respectively, wherein dark lines depict mean ± SEM (**P* < 0.05; Student's *t-*test); **N.** graph of phosphorylated (p) (p-LYN, p-PLCγ2, p-STAT3 and p-ERK1/2) and total (LYN, PLCγ2, STAT3 and ERK1/2) protein levels in immunoblotted protein lysates obtained from isolated leukemia cells from 3 patients with CLL following treatment with 200 nM EC-7072 for 3, 8 and 12 hours; graphs depicting **O.** the mean value of 3 distinct patients normalized to the control (0 hours) condition, whereby levels of phosphorylated and total proteins were quantified by densitometric analysis and the signal intensities were normalized to GAPDH as the control (0 hours) condition, and **P.** the mean value of the densitometry of Western blot analyses of primary CLL cells from 3 different patients normalized to the control (0 hours) condition, whereby signal intensities corresponding to phosphorylated proteins were normalized to their respective total amount for each time point, each following incubation of isolated leukemia cells from 3 patients with CLL after treatment with 200 nM EC-7072 for 3, 8 and 12 hours, and subsequent immunoblotting of protein lysates to detect the indicated proteins (p-LYN, p-PLCγ2, p-STAT3, p-ERK1/2, LYN, PLCγ2, STAT3 and ERK1/2); and **Q.** immunoblots of phosphorylated (p)- and total STAT3 following extraction from MEC-1 cells treated with EC-7072 at the indicated time points.

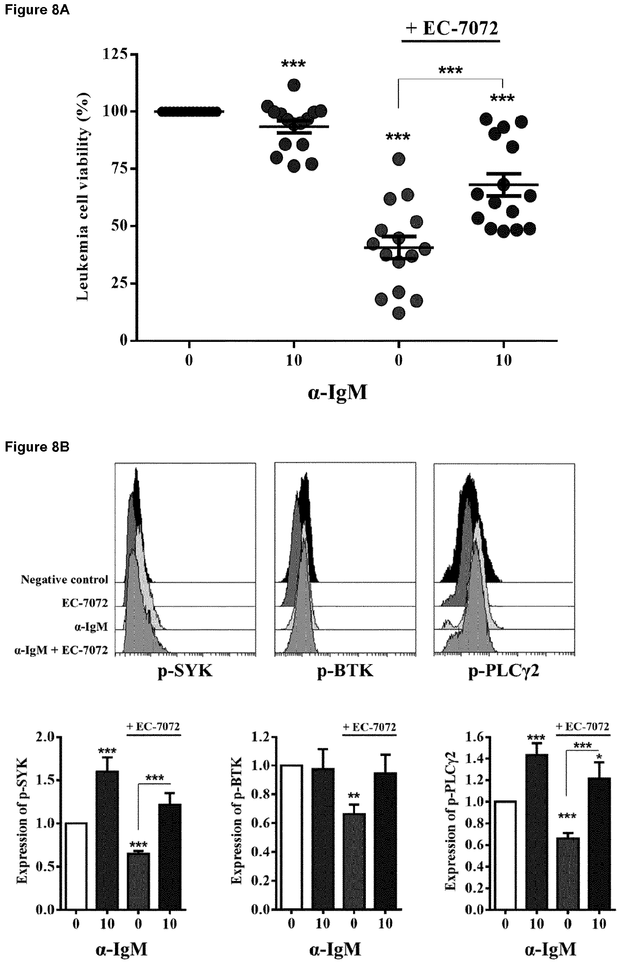

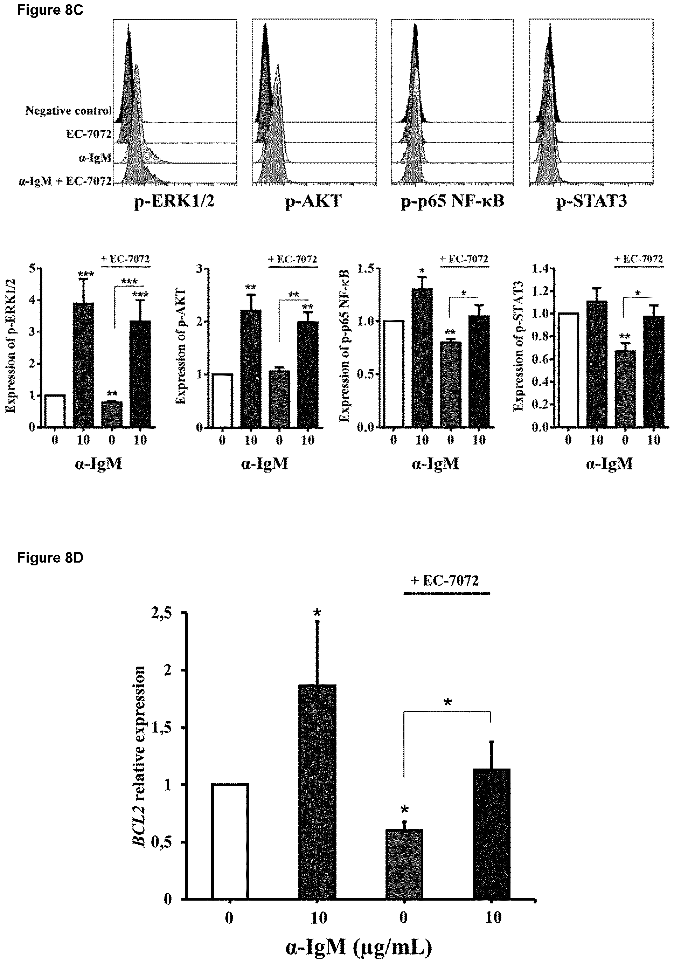

**Figure** 8. Graphs representing **A.** percentage of viable [DiOC₆(3)⁺] cells normalized to their respective control (DMSO) condition for each individual patient whereby leukemia cell viability was determined by cytofluorometric assessment of DiOC₆(3)/PI staining in PBMCs from patients with CLL (n = 15) incubated with EC-7072 (200 nM) in combination with α-IgM (10 µg/mL) for 24 hours, and wherein dark lines represent mean ± SEM; **B.** histograms of the MFI from a representative patient and graphs showing the MFI normalized to the control (DMSO) condition, whereby levels of phosphorylated SYK, BTK and PLCγ2 were analyzed by phosphoflow in isolated leukemia cells from patients with CLL (n = 10) incubated with EC-7072 (200 nM) for 8 hours followed by stimulation with α-IgM (10 µg/mL) for 15 minutes; **C.** histograms of the MFI from a representative patient and graphs showing the MFI normalized to the control (DMSO) condition, whereby levels of phosphorylated ERK1/2, AKT (n = 10), p65 NF-κB and STAT3 (n = 6) were analyzed by phosphoflow in isolated leukemia cells from patients with CLL incubated with EC-7072 (200 nM) for 8 hours followed by stimulation with α-IgM (10 µg/mL) for 15 minutes; and **D.** the mRNA relative expression normalized to the control (DMSO) condition, whereby relative expression of BCL2 was analyzed by qPCR following incubation of isolated leukemia cells from patients with CLL (n = 4) after treatment with EC-7072 (200 nM) in combination with α-IgM (10 µg/mL) for 6 hours and extraction of total RNA. (Mean ± SEM) (*P < 0.05; **P < 0.01; ***P < 0.001; Student's *t*-test).

### Detailed description of the invention

The present invention relates to a compound of Formula (I), as defined herein, or a pharmaceutically acceptable salt, co-crystal or solvate thereof, for use in treatment of a B-cell malignancy.

Thus, the present invention also relates to the use of a compound of Formula (I), as defined herein, or a pharmaceutically acceptable salt, co-crystal or solvate thereof, for the manufacture of a medicament for the treatment of a B-cell malignancy. Likewise, the present invention also relates to a method of treatment of a B-cell malignancy, comprising administering a compound of Formula (I), as defined herein, or a pharmaceutically acceptable salt, co-crystal or solvate thereof, to a subject.

In addition, the present invention relates to a composition comprising:
(a) a compound of Formula (I), as defined herein, or a pharmaceutically acceptable salt, co-crystal or solvate thereof; and
(b) at least one chemotherapeutic agent selected from idelalisib or fludarabine, or a salt, co-crystal or solvate thereof.

Moreover, the present invention relates to a composition comprising:
(a) a compound of Formula (I), as defined herein, or a pharmaceutically acceptable salt, co-crystal or solvate thereof; and
(b) at least one chemotherapeutic agent selected from idelalisib or fludarabine, or a salt, co-crystal or solvate thereof, for use in treatment of disease.

Thus, the present invention also relates to the use of a composition comprising:
(a) a compound of Formula (I), as defined herein, or a pharmaceutically acceptable salt, co-crystal or solvate thereof; and
(b) at least one chemotherapeutic agent selected from idelalisib or fludarabine, or a salt, co-crystal or solvate thereof,
for the manufacture of a medicament for the treatment of a disease. Likewise, the present invention also relates to a method of treatment of a disease, comprising administering a composition comprising:
(a) a compound of Formula (I), as defined herein, or a pharmaceutically acceptable salt, co-crystal or solvate thereof; and
(b) at least one chemotherapeutic agent selected from idelalisib or fludarabine, or a salt, co-crystal or solvate thereof, to a subject.

Moreover, the present invention relates to a composition comprising:
(a) a compound of Formula (I), as defined herein, or a pharmaceutically acceptable salt, co-crystal or solvate thereof; and
(b) at least one chemotherapeutic agent selected from idelalisib or fludarabine, or a salt, co-crystal or solvate thereof, for use in treatment of a B-cell malignancy.

Thus, the present invention also relates to the use of a composition comprising:
(a) a compound of Formula (I), as defined herein, or a pharmaceutically acceptable salt, co-crystal or solvate thereof; and
(b) at least one chemotherapeutic agent selected from idelalisib or fludarabine, or a salt, co-crystal or solvate thereof,
for the manufacture of a medicament for the treatment of a B-cell malignancy. Likewise, the present invention also relates to a method of treatment of a B-cell malignancy, comprising administering a composition comprising:
(a) a compound of Formula (I), as defined herein, or a pharmaceutically acceptable salt, co-crystal or solvate thereof; and
(b) at least one chemotherapeutic agent selected from idelalisib or fludarabine, or a salt, co-crystal or solvate thereof, to a subject.

Additionally, the present invention relates to a method for producing a composition comprising:
(a) a compound of Formula (I), as defined herein, or a pharmaceutically acceptable salt, co-crystal or solvate thereof; and
(b) at least one chemotherapeutic agent selected from idelalisib or fludarabine, or a salt, co-crystal or solvate thereof,
which comprises mixing:
(i) said compound of Formula (I), or a pharmaceutically acceptable salt, co-crystal or solvate thereof; and
(ii) said at least one chemotherapeutic agent selected from idelalisib or fludarabine, or a salt, co-crystal or solvate thereof.

Furthermore, the present invention relates to a kit-of-parts for use in treatment of chronic lymphocytic leukemia, comprising:
(a) a compound of Formula (I), as defined herein, or a pharmaceutically acceptable salt, co-crystal or solvate thereof; and
(b) at least one chemotherapeutic agent selected from idelalisib or fludarabine, or a salt, co-crystal or solvate thereof.

In the present invention, said compound of Formula (I) has the following structure: 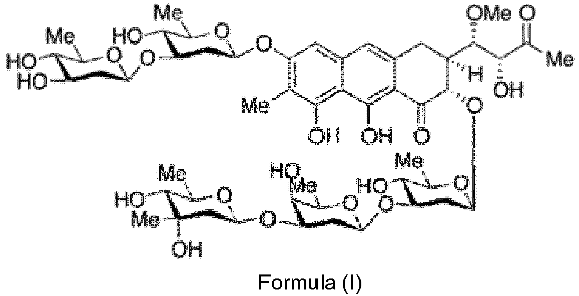 and is a mithramycin analog (mithralog) also known as EC-7072, mithramycin SK or MTM-SK. In the present invention, said compound may be present as Formula (I) or as a pharmaceutically acceptable salt, co-crystal or solvate thereof. Said pharmaceutically acceptable salt is preferably an alkali metal, alkaline earth metal or transition metal salt, more preferably an alkali metal salt selected from a sodium or potassium salt, or an alkaline earth metal salt selected from a calcium or magnesium salt. Said co-crystal or solvate is preferably a co-crystal or solvate of water (i.e. the hydrate), methanol, ethanol, *iso*-propanol, *tert*-butanol, acetone, acetonitrile, chloroform, dichloromethane or ethyl amine, more preferably water. However, preferably said compound is present as Formula (I).

Idelalisib is a phosphoinositide 3-kinase inhibitor, has the following structure: 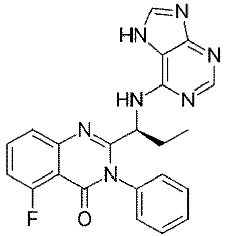 and is sold under the tradename Zydelig for treatment of chronic lymphocytic leukaemia (CLL).

Fludarabine inhibits DNA synthesis, has the following structure: 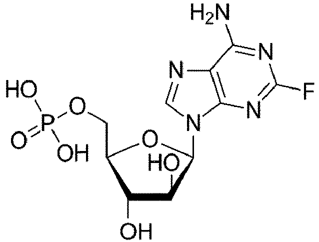 and is sold under the tradename Fludara for treatment of chronic lymphocytic leukaemia (CLL).

B-cell malignancies represent a diverse collection of diseases, and in one embodiment the B-cell malignancy is preferably selected from a non-Hodgkin lymphoma (NHL), a leukaemia or a myeloma. More preferably, the B-cell malignancy is selected from the group comprising chronic lymphocytic leukaemia, follicular lymphoma, non-Hodgkin lymphoma, mantle cell lymphoma and diffuse large B-cell lymphoma. In a preferred embodiment of the invention, the B-cell malignancy is chronic lymphocytic leukaemia (CLL). CLL is identified by progressive accumulation of mature-appearing clonal B cells expressing CD5, CD23 and CD19 surface markers in the blood, bone marrow and secondary lymphatic tissues.

Said B-cell malignancy is comprised in a subject. Preferably said subject is an animal subject, more preferably a mammalian subject, even more preferably a human subject, and yet more preferably an adult human subject. Said adult human subject is preferably over the age of 40 years old, more preferably over the age of 50 years old and even more preferably over the age of 60 years old.

Still more preferably, the B-cell malignancy is chronic lymphocytic leukaemia (CLL) and the subject is a human subject in which CLL has relapsed following prior therapy for said CLL (*i.e*. there is evidence of minimal residual disease or resistance to CLL therapy). Still much more preferably, the B-cell malignancy is chronic lymphocytic leukaemia (CLL) and the subject is a human subject in which IgHV is mutated or is not mutated and in which chromosome 17p and/or chromosome 11q is deleted or is not mutated (*i.e*. said subject having CLL is selected independently of whether IgHV is mutated or not and independently of whether chromosome 17p and/or chromosome 11q is deleted or not).

Thus, said composition may preferably be a pharmaceutical or veterinary composition, but is a pharmaceutical composition when said subject is a human subject.

In a preferred embodiment of those aspects of the present invention relating to:
- the compound of Formula (I), as defined herein, or a pharmaceutically acceptable salt, co-crystal or solvate thereof for use,
- use of a compound of Formula (I), as defined herein, or a pharmaceutically acceptable salt, co-crystal or solvate thereof, and
- method of treatment comprising administering a compound of Formula (I), as defined herein, or a pharmaceutically acceptable salt, co-crystal or solvate thereof,
said compound of Formula (I), or a salt, co-crystal or solvate thereof, is administered simultaneously with, separately from or sequentially to the administration of at least one chemotherapeutic agent selected from idelalisib or fludarabine, or a salt, co-crystal or solvate thereof. Thus, idelalisib, or a salt, co-crystal or solvate thereof, may be administered simultaneously with, separately from or sequentially to said compound of Formula (I) or salt, co-crystal or solvate thereof, meaning that idelalisib, or a salt, co-crystal or solvate thereof, may be administered at the same or a different time as said compound of Formula (I) or salt, co-crystal or solvate thereof, more preferably within 0 and 72 hours of one another. In an even more preferred embodiment, idelalisib is administered 24 hours before said compound of Formula (I). Similarly, fludarabine, or a salt, co-crystal or solvate thereof, may be administered simultaneously with, separately from or sequentially to said compound of Formula (I) or salt, co-crystal or solvate thereof, meaning that fludarabine, or a salt, co-crystal or solvate thereof, may be administered at the same or a different time as said compound of Formula (I) or salt, co-crystal or solvate thereof, more preferably within 0 and 72 hours of one another. In an even more preferred embodiment, fludarabine is administered within 1 hour of said compound of Formula (I).

In a more preferred embodiment of those aspects of the present invention relating to:
- the compound of Formula (I), as defined herein, or a pharmaceutically acceptable salt, co-crystal or solvate thereof for use,
- use of a compound of Formula (I), as defined herein, or a pharmaceutically acceptable salt, co-crystal or solvate thereof, and
- method of treatment comprising administering a compound of Formula (I), as defined herein, or a pharmaceutically acceptable salt, co-crystal or solvate thereof,
wherein said compound of Formula (I), or a salt, co-crystal or solvate thereof, is administered simultaneously with, separately from or sequentially to the administration of at least one chemotherapeutic agent selected from idelalisib or fludarabine, or a salt, co-crystal or solvate thereof, and in a preferred embodiment of those aspects of the present invention relating to:
- the composition,
- the composition for use,
- the use of the composition,
- method of treatment comprising administering the composition,
- the method for producing said composition, and
- the kit-of-parts
the chemotherapeutic agent is idelalisib and the molar ratio of the compound of the Formula (I) to idelalisib is from 1:3 to 1:300, or the chemotherapeutic agent is fludarabine and the molar ratio of the compound of the Formula (I) to fludarabine is from 1:7 to 1:380. Even more preferably, the molar ratio of the compound of the Formula (I) to idelalisib is from 1:4 to 1:200, and/or the molar ratio of the compound of the Formula (I) to fludarabine is from 1:8 to 1:350.

The compound of Formula (I), as defined herein, or a pharmaceutically acceptable salt, co-crystal or solvate thereof may be administered alone or as a composition, where relevant according to the above molar ratios, wherein the amount of the compound of the Formula (I), or a pharmaceutically acceptable salt, co-crystal or solvate thereof, is between 0.001 and 100 mg/kg bodyweight/dose, preferably between 0.01 and 10 mg/kg bodyweight/dose, more preferably between 0.05 and 1 mg/kg bodyweight/dose. Said dose may be administered parenterally (i) in a single daily dose, (ii) divided into n smaller doses which are administered at n intervals throughout the day, whereby n is a number between 2 and 5, preferably between 2 and 3, or (iii) accumulated for m days, whereby said accumulated dose is administered once every m days, whereby m is an interval of between 2 and 28 days, preferably between 3 and 5 days. In an even more preferred embodiment, the compound of Formula (I), as defined herein, or a pharmaceutically acceptable salt, co-crystal or solvate thereof, is administered parenterally as an intravenous injection of an infusion or solution, or subdermal injection of a bolus, infusion or solution (*i.e*. injection as a composition), where relevant according to the above molar ratios, in an amount of between 0.05 and 1 mg/kg bodyweight/dose, once a day, once every three days, twice a week, once weekly, once every fortnight or once every 28 days.

Said compound of Formula (I), as defined herein, or a pharmaceutically acceptable salt, co-crystal or solvate thereof, is preferably comprised in a composition of the present invention and/or a container. In said kit-of-parts, said compound of Formula (I), or a pharmaceutically acceptable salt, co-crystal or solvate thereof and said at least one chemotherapeutic agent are preferably comprised in separate compositions and/or containers.

in a preferred embodiment of those aspects of the present invention relating to:
- the composition of the present invention,
- the composition for use,
- the use of the composition,
- method of treatment comprising administering the composition,
- the method for producing said composition, and
- the kit-of-parts comprising separate compositions,
said compositions preferably each independently comprise an excipient and/or carrier, wherein the excipient and/or carrier is selected from a diluent, bulking agent, filler, anti-adherent, binder, coating, colour, disintegrant, flavour, glidant, lubricant, preservative, sorbent, sweetener or vehicle. Moreover, such compositions can be in crystalline, powder, granular, compacted solid, liquid, solution, suspension, elixir, syrup, emulsion, cream, gel, droplet, mist, vapor or spray form.

Said container is preferably a sealable container selected from a cavity/pocket of a blister pack, capsule, ampoule, sachet, bottle, vial, syringe or nebulizer or combinations thereof, more preferably, said container is a cavity/pocket of a blister pack, a capsule, an ampoule, a bottle or a syringe, furthermore preferably a cavity/pocket of a blister pack, an ampoule or a bottle, most preferably a cavity/pocket of a blister pack, wherein when the compound of Formula (I) and fludarabine or idelalisib, or salts, co-crystals or solvates thereof, are each comprised in separate cavities/pockets of a blister pack, said separate cavities/pockets are part of the same blister pack or part of different blister packs.

### Examples

The following examples illustrate the invention and should not be considered as limiting, but rather illustrative of the invention.

### 1. Materials and methods

### 1.1 Reagents and cell cultures

EC-7072 [the compound of Formula (I)] was provided by EntreChem S.L. (Oviedo, Spain) and made according to the disclosure in J. Am. Chem. Soc. (2003) 125(19):5745-53 and in US patent US7423008 using *Streptomyces argillaceus* through combinatorial biosynthesis by targeted inactivation of a ketoreductase implicated in the biosynthesis of Mithramycin A (MTA). MTA was provided by EntreChem S.L. (Oviedo, Spain) and made according to the disclosure in App. Microbiol. Biotechnol. (2018), 857-869. Other mithralogs, including EC-7073 (also known as MTM-SDK), EC-7092 (also known as demycarosyl-3D-β-D-digitoxosyl-MTM), EC-8011, EC-8041, EC-8042 (also known as demycarosyt-3D-β-D-digitoxosyt-mithramycin SK), EC-13091 and EC-14041 were likewise provided by EntreChem S.L. (Oviedo, Spain). Idelalisib, fludarabine, ibrutinib and venetoclax, as well as Z-VAD-fmk were obtained from Selleckchem. Stock solutions were prepared in dimethyl sulfoxide (DMSO) and stored at -80 °C. DMSO was used as vehicle (control) in all experiments.

Recombinant human (rh) TNF-α, IL-6, IL-4 and BAFF were purchased from Peprotech. Soluble multimeric CD40L was obtained from Adipogen.

All cell lines were obtained from ATCC and cultured in the recommended culture medium supplemented with 10% heat-inactivated FBS (Sigma-Aldrich), 1 mM sodium pyruvate, 2 mM L-glutamine, 100 U/mL penicillin and 10 µg/mL streptomycin at 37 °C and 5% CO₂. HK-2 cells were further supplemented with 36 ng/mL hydrocortisone and Insulin-Transferrin-Selenium (ITS; Gibco). Healthy fibroblasts were obtained from healthy donors and kindly provided by Dr. Isabel Quirós (Universidad de Oviedo, Spain).

### 1.2 Patient samples

Blood samples from untreated patients with CLL (n = 63) were provided by Hospital Universitario Central de Asturias. Written informed consent was obtained from all the patients following the Declaration of Helsinki and samples were collected with approval from the local ethics committee (Comité de Ética de la Investigación del Principado de Asturias, case-19042016). CLL was diagnosed according to standard clinical and laboratory criteria. Buffy-coats from healthy donors (n = 20) were provided by the Centro Comunitario de Sangre y Tejidos de Asturias. Peripheral blood mononuclear cells (PBMCs) were isolated by Ficoll gradient centrifugation from healthy donors and patients with CLL and used fresh. For some experiments, leukemia cells were isolated by using EasySep™ Direct Human B-CLL Cell Isolation Kit (Stemcell Technologies) and T cells were purified using Pan T Cell Isolation Kit (Miltenyi Biotec), following the manufacturer's recommendations. Purity of isolated populations was determined by flow cytometry and only samples with purity higher than 90% were employed. PBMCs and isolated immune subsets were cultured in RPMI 1640 (Lonza) supplemented with 10% heat-inactivated fetal bovine serum (FBS) (Sigma-Aldrich), 1 mM sodium pyruvate, 2 mM L-glutamine, 100 U/mL penicillin and 10 µg/mL streptomycin at 37 °c and 5% CO₂.

### 1.3 Immune cell subset identification

To determine immune cell populations, the following antibodies were employed: anti-CD19-APC, anti-CD3-APC, anti-CD4-PerCP and anti-CD8-APC (all from Immunostep); anti-CD3-FITC and anti-CD56-APC (Cytognos); and anti-CD3-PE/Cy7 and anti-CD5-APC/Cy7 (Biolegend). The subsets of immune cells were identified as follows: T cells were defined as CD3⁺CD56⁻, NK cells were identified as CD3⁻CD56⁺ and healthy B cells as CD19⁺. Leukemia cells were defined as CD19⁺, as the percentage of healthy B cells (CD19⁺CD5⁻) detected in PBMCs from patients with CLL was less than 2% (data not shown). Additionally, antibodies from Biolegend were used to detect surface expression of CD79A (clone: HM47), CD79B (clone: CB3-1) and IgM (clone: MHM-88). Cells were analyzed in a BD FACS Canto II flow cytometer with FACS Diva software (Beckton Dickinson).

### 1.4 Absolute cell number quantification

To evaluate absolute cell numbers, an equal volume of PKH26 reference microbeads (Sigma-Aldrich) was added to each sample after staining for immune subset identification. Microbeads (5x10³) were acquired by flow cytometry and absolute numbers of each immune population within the sample were calculated. Normalized percentage values were calculated considering the control (DMSO)-treated cells as 100%.

### 1.5 Detection of Apoptosis

Cell viability and apoptosis were evaluated by double-staining with the mitochondrial inner transmembrane potential (ΔΨₘ)-sensitive dye DiOC₆(3) (3,3'-dihexyloxacarbocyanine iodide; Sigma-Aldrich) and the exclusion dye propidium iodide (PI; Immunostep), allowing identification of apoptotic [DiOC₆(3)^{low} PI⁻] and dead [PI⁺] cells as per Proc. Natl. Acad. Sci. U.S.A. (2014) 111(8):3020-3025 and Methods Mol. Biol. (2016) 1419:1-16. Briefly, PBMCs were resuspended in culture medium containing 20 nM DiOC₆(3) and incubated for 30 minutes at 37 °C. Then, PI (1 µg/mL) was added and cells were further incubated for 10 minutes at room temperature (RT). Cells were analyzed by flow cytometry considering DiOC₆(3)⁺PI⁻ cells as viable. In some experiments, apoptosis was evaluated by staining with Annexin V/PI. Thus, PBMCs were incubated in Annexin V binding buffer containing 5 µg/mL Annexin V-FITC (Biolegend) for 15 minutes at RT and PI (1 µg/mL) was subsequently added. Data were normalized to the untreated control as a percentage. Only samples with at least 60% of viable cells (DiOC₆(3)⁺; Annexin V⁻) in the control (DMSO) condition were employed in this study.

### 1.6 Caspase-3 activity assay

Intracellular levels of activated caspase-3 were measured by using CaspGLOW™ Fluorescein Active Caspase-3 Staining Kit (Invitrogen). In brief, EC-7072 (200 nM) or DMSO-treated PBMCs from patients with CLL were incubated with FITC-DEVD-fmk for 30 minutes at 37 °c and fluorescence was next analyzed by flow cytometry in leukemia cells. For some experiments, cells were pretreated for 2 hours with the indicated doses of the broad-spectrum caspase inhibitor Z-VAD-fmk.

### 1.7 RNA-seq analysis

Isolated leukemia cells from 8 patients with CLL were incubated with 200 nM EC-7072 or DMSO for 6 hours. Total RNA from isolated CLL cells was extracted using RNeasy Mini-Kit (Qiagen). RNA integrity and concentration were determined using an Agilent-2100 Bioanalyzer (Agilent Technologies) and high-quality RNA samples were further processed. RNA-seq libraries were prepared using TruSeq-Stranded mRNA LT Sample Prep Kit (Illumina) and checked for quality using a DNA 1000 chip on a 2100 Bioanalyzer. Each library was sequenced on a HiSeq 4000 (Illumina) to generate approximately 20 million uniquely-mapping reads per sample. FASTQ files were first evaluated using FastQC for quality control checks (http://www.bioinformatics.babraham.ac.uk/projects/fastqc/). No problems or biases associated to library preparation or sequencing were identified. Transcript abundance was directly quantified with Salmon [Nat. Methods (2017) 14(4):417 - 419], employing the human transcriptome version GRCh38 (Ensembl) as reference. Transcript-level quantifications were aggregated for gene-level differential analysis with DESeq2 as per Genome Biol. (2014) 15(12):550, applying a multifactor design for paired samples. Finally, gene set enrichment analysis was carried out with PathfindR [bioRxiv (2018):272450]. Further gene analysis and representations were performed excluding genes with mean expression lower than 25 in control (DMSO) and EC-7072 conditions.

The RNA-seq datasets generated for this study have been deposited in the Gene Expression Omnibus database (GEO; https://www.ncbi.nlm.nih.gov/geo/) under accession number GSE123777.

### 1.8 Phosphoflow

Protein phosphorylation levels were evaluated by phosphoflow. Briefly, isolated leukemia cells from patients with CLL were treated with 200 nM EC-7072 or DMSO for 8 or 12 hours. In some experiments, BCR signaling was activated with goat F(ab')2 anti-IgM antibody (10 µg/mL; SouthernBiotech) for 45 minutes. Right afterwards, cells were fixed with 1.8% paraformaldehyde for 10 minutes at RT and permeabilized with 90% ice-cold methanol for 25 minutes at 4 °C. Finally, cells were incubated with specific phospho-antibodies (p-SYK, p-BTK, p-ERK1/2 and p-H2AX from Biolegend, and p-PLCγ2, p-AKT, p-p65 NF-κB and p-STAT3 from BD Bioscience) for 30 minutes at RT and mean fluorescence intensity (MFI) was analyzed by flow cytometry.

### 1.9 Western blotting

Isolated CLL cells were treated with 200 nM EC-7072 for 3, 8 and 12 hours and lysed with lysis buffer [50 mM Tris-HCl (pH 8), 150 mM NaCl, 1 mM EDTA, 1% NP-40, 20 mM NaF, EDTA-free protease inhibitor cocktail and phosSTOPTM (Roche)]. After heat denaturation, 25 µg protein were resolved on 10% and 15% SDS-PAGE gels and transferred to PVDF membranes (Sigma-Aldrich). Membranes were blocked with 5% BSA and incubated overnight at 4 °C with the corresponding primary antibodies (p-PLCγ2 (Y759), p-ERK1/2 (T202/Y204), p-STAT3 (Y705), p-LYN (Y507), STAT3 and GAPDH from Cell Signaling, and PLCγ2, ERK1/2, LYN, BCL-xL and Noxa from Santa Cruz Biotechnology). Blots were developed with secondary antibodies conjugated to IRDye680 or IRDye800 (Li-Cor Biosciences) and signal was detected on an Odyssey scanner.

Levels of phosphorylated and total proteins were quantified following Western blotting by densitometric analysis employing ImageJ software (https://imagej.nih.gov/ij/). To determine relative protein amounts, signal intensities were first normalized to GAPDH and the ratio between phosphorylated and total proteins was then calculated.

### 1.10 Intracellular staining

PBMCs from patients with CLL were treated with 200 nM EC-7072 or DMSO for 24 hours and stimulated with PMA (50 ng/mL) and ionomycin (1 µg/mL) for 5 hours in the presence of GolgiPlug protein transport inhibitor (Beckton Dickinson). T cells and NK cells were then analyzed for intracellular levels of IFN-γ and perforin by flow cytometry.

### 1.11 qPCR analysis

Isolated leukemia cells from patients with CLL were incubated with EC-7072 (200 nM) or DMSO for 6 hours. Total RNA was extracted using RNeasy Mini Kit (Qiagen). 2 µg total RNA were used for cDNA synthesis using High-Capacity cDNA Reverse Transcription Kit (Applied Biosystems). Quantitative RT-PCR was performed using SYBR Green PCR Master Mix (Applied Biosystems) in a 7300 Real-Time PCR System and each sample was analyzed in triplicate. Primers used for each gene detection are provided in **Table 1.**

**Table 1. Primers used in qPCR**

| **Gene** | **Primer sequence 5'-3'** | **Identifier** | **Sense** |
|---|---|---|---|
| *CD79B* | CCAGGCTGGCGTTGTCTCCTG | SEQ ID NO:1 | Forward |
| | GGTACCGGTCCTCCGATCTGGC | SEQ ID NO:2 | Reverse |
| *SYK* | TGCACTATCGCATCGACAAAG | SEQ ID NO:3 | Forward |
| | CATTTCCCTGTGTGCCGATTT | SEQ ID NO:4 | Reverse |
| *LYN* | ACCAGGGAGGAGCCCATTTA | SEQ ID NO:5 | Forward |
| | CTTCCGCTCGATGTATGCCA | SEQ ID NO:6 | Reverse |
| *PIK3CD* | ACTCTGCCATTGTCTAAGCCACCT | SEQ ID NO:7 | Forward |
| | TCACAGCAGGTTCCCAAAGGTGAT | SEQ ID NO:8 | Reverse |
| *PLCG2* | GCCTGTCCCTTTGTAGAAGTGG | SEQ ID NO:9 | Forward |
| | GGCCATTATCATTCACAACCG | SEQ ID NO:10 | Reverse |
| *BCL2* | CTGCACCTGACGCCCTTCACC | SEQ ID NO:11 | Forward |
| | CACATGACCCCACCGAACTCAAAGA | SEQ ID NO:12 | Reverse |
| *BCL2L11* | GGCCCCTACCTCCCTACA | SEQ ID NO:13 | Forward |
| | GGGGTTTGTGTTGATTTGTCA | SEQ ID NO:14 | Reverse |
| *BID* | CGCACCTACGTGAGGAGCTTAGCC | SEQ ID NO:15 | Forward |
| | TGACCACATCGAGCTTTAGCCAGTCA | SEQ ID NO:16 | Reverse |
| *BCL2L1* | GATCCCCATGGCAGCAGTAAAGCAAG | SEQ ID NO:17 | Forward |
| | CCCCATCCCGGAAGAGTTCATTCACT | SEQ ID NO:18 | Reverse |
| *PMAIP1* | CAGAGCTGGAAGTCGAGTGT | SEQ ID NO:19 | Forward |
| | AGGAGTCCCCTCATGCAAGT | SEQ ID NO:20 | Reverse |
| *CARD11* | TTGTGGGAGAATGTGGAGTGT | SEQ ID NO:21 | Forward |
| | TGCCCCTTGGTATGTAGAATG | SEQ ID NO:22 | Reverse |
| *B2M* | TGCTGTCTCCATGTTTGATGTATCT | SEQ ID NO:23 | Forward |
| | TCTCTGCTCCCCACCTCTAAGT | SEQ ID NO:24 | Reverse |

### 1.12 Statistical analysis

Sample size was based on the number of patients or volunteers enrolled to previously designed studies. Statistical analyses were performed using GraphPad Prism 6 software (GraphPad Software Inc.) and appropriate statistical tests were applied to each experiment. For all tests, differences were considered statistically significant for p-values: *P* < 0.05 (*), *P* < 0.01 (**), *P* < 0.001 (***).

For drug combination studies, synergy was evaluated using CalcuSyn Version 2.0 software (Biosoft) that allows the calculation of the combination index (CI) based on the algorithm reported in Cancer Res. (2010) 70(2):440-446. CI values < 0.9 represent synergistic effect, values between 0.9 and 1.1 indicate additive effect and values > 1.1 represent an antagonistic effect. CI values for non-fixed ratio combinations from independent experiments were generated and plotted.

### 2. EC-7072 has a cytotoxic effect against primary leukemia cells from patients with CLL independently from cytogenetic aberrations and IGHV mutational status

### 2.1 Methodology

The *in vitro* cytotoxic activity of EC-7072 against primary CLL cells was assessed by:
(i) incubating PBMCs isolated from patients with CLL (n = 13) following treatment with increasing concentrations of EC-7072 (0 - 400 nM) for 24 hours. Numbers of leukemia cells were evaluated by flow cytometry and normalized to their respective control (DMSO) condition for each individual patient to obtain percentage of leukemia cells **(****Figure 1A****)**;
(ii) incubating PBMCs from patients with CLL (n = 10) with or without EC-7072 (100-400 nM) for 24 hours. Viability of leukemia cells was determined by cytofluorometric assessment of DiOC₆(3)/PI staining and normalized to their respective control condition to obtain percentage viability **(****Figure 1B****);**
(iii) incubating PBMCs from patients with CLL (n = 10) with increasing concentrations of EC-7072 (0 - 400 nM) for 24 hours. Apoptosis of leukemia cells was determined by DiOC₆(3)/PI staining and flow cytometry to obtain the mean percentages of apoptotic [DiOC₆(3)^{low} PI⁻] and dead [PI⁺] cells **(****Figure 1C****);**
(iv) incubating PBMCs from patients with CLL (n = 10) with or without EC-7072 (200 nM) for 12 to 72 hours. Viability of leukemia cells was determined by DiOC6(3)/PI staining and normalized to their respective control condition to obtain percentage viability **(****Figure 1D****);**
(v) incubating PBMCs from patients with CLL (n = 24) with MTA (200 nM) or EC-7072 (200 nM) for 24 hours. Leukemia cell death was determined by DiOC₆(3)/PI staining and flow cytometry to obtain the mean percentages of apoptotic [DiOC₆(3)^{low} PI⁻] and dead [PI⁺] cells **(****Figure 1E****);**
(vi) incubating PBMCs from patients with CLL (n = 63) or healthy donors (n = 20) with or without EC-7072 (200 nM) for 24 hours. Viability of leukemia cells and healthy B cells was evaluated by DiOC₆(3)/PI staining and normalized to their respective control condition to obtain percentage viability **(****Figure 1F****)**;
(vii) incubating PBMCs from patients with CLL (n = 62) following treatment with EC-7072 (200 nM) for 24 hours and obtaining a cytogenetic profile for each patient. Viability of leukemia cells was evaluated by DiOC₆(3)/PI staining and normalized to their respective control (DMSO) condition for each individual patient to obtain the percentage of viable [DiOC₆(3)⁺] cells **(****Figure 1G****)**;
(viii) incubating PBMCs from patients with CLL (n = 59) following treatment with EC-7072 (200 nM) for 24 hours and determining mutational status of IgHV for each patient. Viability of leukemia cells was evaluated by DiOC₆(3)/PI staining and normalized to their respective control (DMSO) condition for each individual patient to obtain the percentage of viable [DiOC₆(3)⁺] cells **(****Figure 1H****).**

### 2.2 Results

Treatment of PBMCs isolated from CLL patients with increasing concentrations of EC-7072 significantly decreased leukemia cell numbers and viability in a dose- **(****Figures 1A, 1B** and **1C****)** and a time- **(****Figure 1D****)** dependent manner, these results demonstrating that EC-7072 exerts potent antileukemic *in vitro* activity in CLL at nanomolar concentrations. The antileukemic activity of EC-7072 was comparable to that of MTA **(****Figure 1E****).** In addition, EC-7072 triggered death of normal B cells from healthy volunteers, which, in addition to its lethal effect on CLL cells, suggests that the compound displays high cytotoxicity against B cells (**Figure 1F**)**.** The ability of EC-7072 to induce death of both non-malignant and leukemia B cells underscores a selectivity of the compound to target this immune cell subset.

Moreover, the cytotoxic effect of EC-7072 was not affected by the presence of chromosomal and molecular genetic alterations that are markers of patient prognosis and therapy response in CLL according to the iwCLL guidelines for diagnosis, indications for treatment, response assessment, and supportive management of CLL, as taught in Blood (2018) 131(25):2745-60. Noteworthy is that no differences in the sensitivity to EC-7072 were observed among patients carrying diverse cytogenetic aberrations that define high-risk CLL, including those mediating apoptotic responses to genotoxic compounds, such as del(17p) or del(11q) **(****Figure 1G****).** Likewise, the mutational status of IgHV did not significantly affect the sensitivity to EC-7072 in patients with CLL **(****Figure 1H****).** Overall, these findings support that the mithralog EC-7072 exerts a strong *ex vivo* antileukemic activity against primary cells from patients with CLL, including those bearing genetic markers typically associated to clinical resistance to current chemotherapy modalities.

### 3. EC-7072 does not markedly alter the homeostasis of healthy immune cells from patients with CLL

### 3.1 Methodology

The effect of EC-7072 toward healthy immune subsets, T lymphocytes and natural killer (NK) cells, which are chronically exposed to leukemia cells in the peripheral blood of patients with CLL was assessed by:
(i) incubating PBMCs isolated from patients with CLL (n = 10) following treatment with increasing concentrations of EC-7072 (0 - 400 nM) for 24 hours. Numbers of T cells (CD3⁺CD56⁻) **(****Figure 2A****)** and NK cells (CD3⁻CD56⁺) **(****Figure 2B****)** were evaluated by flow cytometry and normalized to their respective control (DMSO) condition for each individual patient to obtain percentage of relative number of T cells and NK cells, respectively;
(ii) incubating PBMCs isolated from patients with CLL (n = 6 - 10) following treatment with increasing concentrations of EC-7072 (0 - 400 nM) for 24 hours. Viability of immune subsets (leukemia cells, T cells and NK cells) was determined by cytofluorometric assessment of DiOC₆(3)/PI staining and normalized to their respective control (DMSO) condition for each individual patient to obtain percentage of viable [DiOC₆(3)⁺] cells **(****Figures 2C** and **2D****)**;
(iii) incubating PBMCs isolated from patients with CLL (n = 11) following treatment with DMSO or EC-7072 (200 nM) for 24 hours. Numbers of CD4⁺ cells **(****Figure 2E****)** and CD8⁺ T cells **(****Figure 2F****)** were evaluated by flow cytometry and normalized to their respective control (DMSO) condition for each individual experiment;
(iv) incubating PBMCs isolated from healthy donors (n = 15) following treatment with EC-7072 (200 nM) for 24 hours. Numbers of T cells and NK cells were evaluated by flow cytometry and normalized to their respective control (DMSO) condition for each individual experiment **(****Figure 2G****)**, while viability of immune subsets (T cells and NK cells) was determined by cytofluorometric assessment of DiOC₆(3)/PI staining and the percentage of viable [DiOC₆(3)⁺] cells was normalized to their respective control (DMSO) condition (**Figure 2H**);
(v) incubating PBMCs isolated from patients with CLL (n = 4) following treatment with EC-7072 (200 nM) for 24 hours and evaluating IFN-γ and perforin expression upon PMA/ionomycin stimulation in T cells and NK cells by intracellular flow cytometry to obtain the percentage of IFN-γ⁺ cells (**Figure 2I**) or perforin⁺ cells (**Figure 2J**), respectively;
(vi) incubating PBMCs (**Figure 2K**) and leukemia cells (**Figure 2L**) isolated from patients with CLL (n = 24) following treatment with EC-7072 (200 nM) for 24 hours. Apoptosis was examined by DiOC₆(3)/PI staining for each individual patient to obtain percentages of viable [DiOC₆(3)⁺], apoptotic [DiOC₆(3)^{low} PI⁻] and dead [PI⁺] cells;
(vii) incubating PBMCs isolated from patients with CLL (n = 10) following treatment with MTA (200 nM), EC-7072 (200 nM) or other mithralogs (EC-7073, EC-7092, EC-8011, EC-8041, EC-8042, EC-13091 or EC-14041, each at 200 nM) for 24 hours. Viability of leukemia cells **(****Figure 2M****)** was evaluated by flow cytometry and normalized to the respective control (DMSO) condition for each individual experiment to obtain percentage viability;
(viii) incubating PBMCs isolated from healthy donors (n = 10) following treatment with MTA (200 nM), EC-7072 (200 nM) or other mithralogs (EC-7073, EC-7092, EC-8011, EC-8041, EC-8042, EC-13091 or EC-14041, each at 200 nM) for 24 hours. Viability of T cells **(****Figure 2N****)** and NK cells **(****Figure 2O****)** was evaluated by flow cytometry and normalized to the respective control (DMSO) condition for each individual experiment to obtain percentage viability;
(ix) incubating PBMCs from patients with CLL (n = 6) with MTA (200 nM), EC-7072 (200 nM) or other mithralogs (EC-7073, EC-7092, EC-8011, EC-8041, EC-8042, EC-13091 or EC-14041, each at 200 nM) for 24 hours. Leukemia cell death was determined by DiOC₆(3)/PI staining and numbers of CD19⁺ cells were evaluated by flow cytometry to obtain the mean percentages of apoptotic [DiOC₆(3)^{low} PI⁻] and dead [PI⁺] cells **(****Figure 2P****);**
(x) incubating PBMCs isolated from healthy donors (n = 4) following treatment with MTA (200 nM), EC-7072 (200 nM) or EC-7073 (200 nM) for 24 hours. Leukemia cell death was determined by DiOC₆(3)/PI staining and numbers of T cells **(****Figure 2Q****)** and NK cells **(****Figure 2R****)** were evaluated by flow cytometry to obtain the mean percentages of apoptotic [DiOC₆(3)^{low} PI⁻] and dead [PI⁺] cells.

### 3.2 Results

Treatment of PBMCs from patients with CLL with increasing doses of EC-7072 exerted negligible effects on T cell numbers **(****Figure 2A****)** and viability **(****Figures 2C** and **2D****),** finding no differences between CD4⁺ and CD8⁺ T cell subpopulations **(****Figures 2E** and **2F****).** Likewise, NK cell survival was only slightly altered by exposure to 200 nM of EC-7072, a concentration of the mithralog that dramatically induced CLL cell death **(****Figure 1B****)**; and even high doses (400 nM) of the compound were significantly less toxic against NK cells than leukemia cells **(****Figures 2B, 2C** and **2D****).** Thus, treatment of PBMCs from patients with CLL revealed significantly higher cytotoxicity of EC-7072 against leukemia cells compared to circulating NK cells and, above all, T cells. All subsequent experiments were therefore performed with 200 nM of EC-7072, unless otherwise indicated. Exposure of PBMCs from healthy donors to EC-7072 rendered comparable effects on cell numbers and viability of T lymphocytes and NK cells, reinforcing the notion that the compound is clearly less toxic against these immune subsets (**Figures 2G** and **2H**)**.** Thus, EC-7072 is substantially less toxic than the parental compound against human healthy cells of different tissue origins.

Remarkably, the production of the key antitumor effector molecules interferon (IFN)-γ and perforin by T cells and NK cells was not significantly altered upon treatment with the mithralog, suggesting that the antitumor function of immune effector cells is not impaired by EC-7072 (**Figures 2I** and **2J**) and thus EC-7072 does not exacerbate the immune dysregulation frequently observed in patients with CLL. Thus, to test whether the observed leukemia cell death induced by this compound was mediated by the immune cells present in the environment of peripheral CLL cells, we evaluated the effect of EC-7072 on isolated leukemia cells from patients with CLL. As shown in **Figures 2K** and **2L****,** the potent antileukemic activity of EC-7072 on PBMCs from patients with CLL was mimicked when isolated leukemia cells were used, demonstrating that the mithralog exerts its cytotoxic effect on leukemia cells independently of the activity of immune effector cells (i.e. cell death was observed when isolated CLL cells were exposed to EC-7072 in the absence of other immune cell subsets, indicating that the mithralog directly elicits leukemia cell demise).

Moreover, EC-7072 was found to provide effective treatment of CLL, second only to the mithralog EC-7073 **(****Figure 2M****),** yet unlike this mithralog to not exhibit toxicity against T cells or NK cells **(****Figures 2N** and **2O****).** This effectiveness of EC-7072 in the treatment of CLL without affecting other lymphocytic populations was found to be due to the fact that it induces a higher ratio of apoptosis in CLL cells relative to CLL cell death than other mithralogs such as EC-7073 (with the exception of the less effective EC-14041 mithralog, **Figure 2P****),** yet not only induces a ratio of apoptosis to cell death in T cells which is comparable to that induced by EC-7073 **(****Figure 2Q****),** but also induces a far lower ratio of apoptosis in NK cells relative to NK cell death than does EC-7073 **(****Figure 2R****).**

### 4. EC-7072 induces caspase-dependent apoptosis in primary CLL cells that is not counteracted by microenvironmental supportive stimuli.

### 4.1 Methodology

The induction of caspase-dependent apoptosis on primary CLL cells by EC-7072 was assessed by:
(i) incubating PBMCs isolated from patients with CLL (n = 6) following treatment with increasing concentrations of EC-7072 (0 - 400 nM) for 24 hours. Apoptosis of leukemia cells was evaluated by cytofluorometric assessment of Annexin V/PI staining to obtain percentages of apoptotic [Annexin V⁺ PI⁻] and dead [PI⁺] cells **(****Figure 3A****)**;
(ii) incubating PBMCs and T cells isolated from patients with CLL (n = 3) following treatment with EC-7072 (200 nM) for 24 hours. T cell apoptosis was evaluated by cytofluorometric assessment of Annexin V/PI staining to obtain percentages of viable [Annexin V-], apoptotic [Annexin V⁺ PI⁻] and dead [PI⁺] cells **(****Figure 3B****)**;
(iii) incubating PBMCs isolated from patients with CLL (n = 6) following treatment with EC-7072 (0 - 400 nM) for 24 hours. Levels of active caspase-3 in leukemia cells were assessed by flow cytometry to obtain mean fluorescence intensity (MFI) **(****Figure 3C****)** before being normalized to the control (DMSO) condition (**Figure 3D**);
(iv) incubating leukemia cells isolated from patients with CLL (n = 4) following treatment with EC-7072 (200 nM) for 12 hours. Levels of phosphorylated H2AX were assessed by phosphoflow to obtain MFI before being normalized to the control (DMSO) condition (**Figure 3E**);
(v) incubating PBMCs isolated from patients with CLL (n = 6) which had been pretreated with increasing doses of caspase inhibitor Z-VAD-fmk (0-100 µM) followed by exposure to EC-7072 (200 nM) for 24 hours. Viability of leukemia cells was determined by DiOC₆(3)/PI staining and normalized to their respective control (DMSO) condition to obtain percentage of viable [DiOC₆(3)⁺] leukaemia cells **(****Figure 3F****)**, while levels of active caspase-3 were examined by flow cytometry in leukemia cells to obtain MFI before being normalized to the control (DMSO) condition **(****Figure 3G****)**;
(vi) incubating PBMCs isolated from patients with CLL (n = 7) following treatment with EC-7072 (200 nM) in combination with CD40L (100 ng/mL), BAFF (50 ng/mL), TNF-α (40 ng/mL), IL-6 (40 ng/mL) or IL-4 (40 ng/mL) for 24 hours. Viability of leukemia cells was assessed by DiOC₆(3)/PI staining and normalized to their respective control (DMSO) condition for each individual patient to obtain percentage of viable [DiOC₆(3)⁺] leukaemia cells **(****Figure 3H****)**;
(vii) incubating PBMCs isolated from patients with CLL (n = 7) which were co-cultured with OP9 cells for 72 hours, following treatment with EC-7072 (200 nM) for 24 hours. Viability of leukemia cells was assessed by DiOC₆(3)/PI staining and normalized to their respective control (DMSO) condition for each individual patient to obtain percentage of viable [DiOC₆(3)⁺] leukaemia cells **(****Figure 3I****).**

### 4.2 Results

Experiments performed with DiOC₆(3)/PI evinced a compromised leukemia cell plasma membrane integrity and mitochondrial transmembrane potential, which are apoptosis-related parameters, upon EC-7072 treatment. These results were further verified by Annexin V/PI flow cytometric analyses showing that EC-7072 significantly induces CLL cell apoptosis **(****Figure 3A****).** Conversely, no significant apoptosis was detected in T cells isolated from patients with CLL cultured in the presence of EC-7072, further supporting the notion that the mithralog displays selective toxicity against B cells compared to other immune cells **(****Figure 3B****).**

Treatment of CLL cells with EC-7072 resulted in a dose-dependent increase of the intracellular levels of active caspase-3 **(****Figures 3C** and **3D****)** and heightened phosphorylation of histone H2AX **(****Figure 3E****),** which constitutes a cellular response triggered by caspase activation during apoptosis, suggesting that EC-7072 may promote leukemia cell death through a caspase-dependent pathway. Indeed, treatment with increasing doses of the broad-spectrum caspase inhibitor Z-VAD-fmk completely abrogated the death of primary CLL cells induced by the mithralog **(****Figure 3F****).** As expected, the protective effect of Z-VAD-fmk correlated with a decrease of active caspase-3 levels on leukemia cells, thus reinforcing the specificity of the assay **(****Figure 3G****).**

A hallmark of CLL is the strong dependence of leukemia cells for prosurvival and growth signals provided by the microenvironment. Thus, it was investigated whether exposure to CD40 ligand (CD40L), B-cell activating factor (BAFF), tumor necrosis factor (TNF)-α, interleukin (IL)-6 or IL-4, which are microenvironment-derived cytokines reported to enhance CLL viability and drug resistance, could subvert the activity of EC-7072. As shown in **Figure 3H****,** none of the soluble factors tested significantly affected the leukemia cell death brought about by the mithralog. Furthermore, co-culture of PBMCs from patients with CLL in the presence of OP9 stromal cells to mimic a protective microenvironmental niche did not enhance leukemia cell viability upon EC-7072 treatment (**Figure 3I**)**.** Exposure of OP9 cells to EC-7072 for 24 hours did not affect their viability. Thus, in spite of chronic activation of the BCR pathway observed in several B-cell malignancies, the exposure to microenvironment-derived factors, as well as co-culture with stromal cells, which are known to mediate therapy resistance in patients with CLL, failed to counteract the antileukemic activity of EC-7072, hence suggesting that the efficacy of the compound does not diminish *in vivo.*

A major pitfall of CLL therapies is the development of therapeutic resistance in high-risk patients. Of relevance, CLL cell death induced by EC-7072 was not affected by the presence of molecular and cytogenetic aberrations found in patients with CLL refractory to certain chemotherapeutic strategies or the use of soluble microenvironment-derived factors that sustain leukemia cell survival.

Altogether, these results indicate that the cytotoxic activity of EC-7072 against primary CLL cells entails caspase activation and that this antileukemic activity is not prevented by microenvironmental stimulatory signals.

### 5. EC-7072 displays a cytotoxic activity against primary CLL cells analogous and additive with that of therapies routinely used in CLL.

### 5.1 Methodology

The *ex vivo* antileukemic efficacy of EC-7072 was evaluated to determine whether it is comparable to that of therapies currently approved for patients with CLL. In particular, *the ex vivo* antileukemic efficacy of EC-7072 was compared against other therapies by:
(i) incubating PBMCs isolated from patients with CLL (n = 4 - 6) following treatment with fludarabine, venetoclax or EC-7072 for 24 hours, or ibrutinib or idelalisib for 48 hours. CLL cell viability was assessed by flow cytometric detection of DiOC₆(3)/PI staining and normalized to their respective control (DMSO) condition for each individual patient to obtain percentage of surviving [DiOC₆(3)⁺] cells (**Figure 4A**);
(ii) incubating PBMCs isolated from patients with CLL (n = 6) following exposure to non-fixed ratio combinations of EC-7072 at increasing concentrations with fludarabine at concentrations corresponding to IC₁₀, IC₂₅ and IC₅₀ values calculated therefor for 24 hours (**Figure 4B**) or idelalisib at concentrations corresponding to IC₁₀ and IC₂₅ values calculated therefor for 48 hours (**Figure 4C**)**.** Leukaemia cell viability was assessed by DiOC₆(3)/PI staining and normalized to their respective control (DMSO) condition for each individual patient to obtain percentage of viable [DiOC₆(3)⁺] cells. Cl values were generated for each combination of EC-7072 with fludarabine (**Figure 4D** and **Table 2)** or idelalisib (**Figure 4E** and **Table 3**) using CalcuSyn software according to the Chou-Talalay method, wherein Cl values < 0.9 indicate synergism, values between 0.9 - 1.1 correspond to additive effects and values > 1.1 represent antagonism, and the fraction affected (Fa) was calculated based on the percentage of viable cells after the treatment;
(iii) incubating PBMCs isolated from patients with CLL (n = 6) following treatment with idelalisib at concentrations corresponding to IC₁₀ and IC₂₅ values calculated therefor for 48 hours, wherein non-fixed ratio combinations of EC-7072 at increasing concentrations were added to the cell culture for the last 24 hours of incubation. Leukaemia cell viability was assessed by DiOC₆(3)/PI staining and normalized to their respective control (DMSO) condition for each individual patient to obtain percentage of viable [DiOC₆(3)⁺] cells. CI values were generated for each combination of EC-7072 with idelalisib using CalcuSyn software according to the Chou-Talalay method, wherein CI values < 0.9 indicate synergism, values between 0.9 - 1.1 correspond to additive effects and values > 1.1 represent antagonism, and the fraction affected (Fa) was calculated based on the percentage of viable cells after the treatment (**Table 4**);
(iv) incubating PBMCs isolated from patients with CLL (n = 6 - 8) following treatment with fludarabine (10 µM) for 24 hours (**Figure 4F**) or idelalisib (10 µM) for 48 hours (**Figure 4G**), wherein EC-7072 (200 nM) was added to the cell culture for the last 24 hours of incubation. Viability of immune subsets (T cells and NK cells) was assessed by DiOC₆(3)/PI staining and normalized to their respective control (DMSO) condition for each individual patient to obtain percentage of viable [DiOC₆(3)⁺] cells.

### 5.2 Results

Acquired resistance to targeted CLL therapies is frequently conferred by mutations in genes encoding BCR signaling mediators, with co-occurring mutations in BTK and PLCG2 having been shown to mediate ibrutinib resistance, while a point mutation in BCL2 has recently been identified in patients with CLL resistant to treatment with venetoclax. However, EC-7072 displays comparable or higher killing activity on CLL cells than that of fludarabine, ibrutinib, idelalisib and venetoclax, used at previously reported doses. When PBMCs from patients with CLL were treated with EC-7072, fludarabine or the targeted agents ibrutinib, idelalisib or venetoclax in a range of doses and leukemia cell viability was assessed by DiOC₆(3)/PI staining and flow cytometric detection, it was found that treatment with EC-7072 exerted similar cytotoxicity against primary CLL cells to that of ibrutinib or venetoclax, but reduced CLL cell viability to a greater extent than idelalisib or fludarabine under the experimental settings tested (**Figure 4A**). To determine the combined efficacy, PBMCs from patients with CLL were incubated with non-fixed ratio combinations of EC-7072 with fludarabine or idelalisib at IC₁₀, IC₂₅ and IC₅₀ doses, whereby EC-7072 enhanced the effect of the these two therapeutic agents tested in killing primary CLL cells (**Figures 4B** and **4C**) and calculation of CI values unraveled that the combination of EC-7072 with idelalisib or fludarabine exerts synergistic cytotoxicity on leukemia cells (**Figures 4D** and **4E** and **Tables 2** and **3**). The synergistic effect observed upon combination with idelalisib suggests that both compounds might be targeting distinct signaling pathways, which would correlate with EC-7072 not likely inhibiting AKT signaling.

**Table 2. Svneraic combinations of EC-7072 and fludarabine**

| **[EC-7072] 24 h (nM)** | **[Fludarabine] 24 h (nM)** | **Molar ratio EC-7072:Fludarabine** | **Combination Index** |
|---|---|---|---|
| 50 | 1000 | 1:20.00 | 0.61 |
| 50 | 5000 | 1:100.00 | 0.48 |
| 50 | 19000 | 1:380.00 | 0.38 |
| 80 | 1000 | 1:12.50 | 0.74 |
| 80 | 5000 | 1:62.50 | 0.55 |
| 80 | 19000 | 1:237.50 | 0.44 |
| 130 | 1000 | 1:7.69 | 0.87 |
| 130 | 5000 | 1:38.46 | 0.63 |
| 130 | 19000 | 1:146.15 | 0.60 |

**Table 3. Synergic combinations of EC-7072 and idelalisib (concurrent treatment)**

| **[EC-7072] 48 h (nM)** | **[Idelalisib] 48 h (nM)** | **Molar ratio EC-7072:Idelalisib** | **Combination Index** |
|---|---|---|---|
| 30 | 400 | 1:13.33 | 0.59 |
| 30 | 9000 | 1:300.00 | 0.44 |
| 60 | 400 | 1:6.67 | 0.63 |
| 60 | 9000 | 1:150.00 | 0.42 |
| 100 | 400 | 1:4.00 | 0.73 |
| 100 | 9000 | 1:90.00 | 0.50 |

In **Table 3**, idealisib was tested in combination with EC-7072 over 48 h of incubation, however in **Table 4** cells were exposed to idelalisib for 48 h but to EC-7072 for only 24 h (the last 24 h of exposure to idelalisib). Both experiments provided evidence of synergy, the former additionally providing evidence a concurrent treatment and the latter providing evidence of a sequential treatment.

**Table 4. Synergic combinations of EC-7072 and idelalisib (sequential treatment)**

| **[EC-7072] 24 h (nM)** | **[Idelalisib] 48h (nM)** | **Molar ratio EC-7072:Idelalisib** | **Combination Index** |
|---|---|---|---|
| 50 | 400 | 1:8 | 0.47 |
| 50 | 9000 | 1:180.00 | 0.38 |
| 80 | 400 | 1:5.00 | 0.59 |
| 80 | 9000 | 1:112.50 | 0.29 |
| 130 | 400 | 1:3.08 | 0.57 |
| 130 | 9000 | 1:69.23 | 0.34 |

Additionally, the impact of the combined treatments on the survival of healthy immune subsets, T cells and NK cells, was evaluated, showing that EC-7072 was significantly less toxic than fludarabine against T cells and NK cells from patient's PBMCs (**Figure 4F**)**.** Idelalisib exerted negligible effects on the viability of these populations, similarly to EC-7072 (**Figure 4G**). Of note, the combination treatment resulted in incremented toxicity against NK cells (**Figures 4F** and **4G**). Thus, although synergistic activity of EC-7072 with fludarabine or idelalisib was observed, EC-7072 was significantly less toxic to non-malignant immune cells from the same patients than other agents.

Collectively, these assays indicate that EC-7072 displays analogous or higher *ex vivo* antileukemic activity than that of drugs currently employed for CLL therapy and shows an additive or synergistic effect with these agents, suggesting that the use of this mithralog alone or in combination regimens may represent a novel therapeutic approach for CLL.

### 6. EC-7072 does not markedly affect non-tumoral cells but is effective against different B-cell malignancies

### 6.1 Methodology

Effectiveness of EC-7072 against different B-cell malignancies was assessed by;
(i) incubating primary fibroblasts from healthy donors (n = 4) and HK-2 cells from healthy human adult kidney (n = 3) following treatment with MTA (200 nM) or EC-7072 (200 nM) for 24 hours. Cell death was evaluated by PI staining to obtain the percentages corresponding to the [PI⁺] subset (**Figure 5A**) and the mean percentage of [PI⁺] cells (**Figure 5B**);
(ii) treating MEC-1, GRANTA-519, Z-138, JVM-2, CA-46, Ramos, Raji and U266 cell lines (n = 3) following treatment with EC-7072 (500 nM) followed by incubation for 24 hours. Cell viability was assessed by DiOC₆(3)/PI staining and normalized to control (DMSO) condition to obtain mean percentage of viable [DiOC₆(3)⁺] cells (**Figure 5C**)**.**

### 6.2 Results

In sharp contrast with the high toxicity of MTA, EC-7072 did not markedly affect the viability of non-tumoral cells such as primary human fibroblasts and an immortalized cell line derived from normal adult human kidney (HK-2) (**Figures 5A** and **5B**). In other words, EC-7072 is substantially less toxic than MTA against human healthy cells of different tissue origins. In agreement, EC-7072 significantly reduced the surviving cell fraction of a panel of tumor cell lines derived from B-cell hematologic malignancies (**Figure 5C**). Thus, although EC-7072 does not markedly affect non-tumoral cells it is effective against different B-cell malignancies.

### 7. EC-7072 modulates the transcriptome and dysregulates BCL2 expression in primary CLL cells.

### 7.1 Methodology

Isolated leukemia cells from patients with CLL (n = 8) were treated with 200 nM EC-7072 for 6 hours. Total RNA was extracted and RNA-seq analysis was performed. Heat maps were generated to identify:
(i) statistically significant differences in gene expression between negative control (DMSO) and EC-7072-treated samples using a scale representing the per-gene Z-score and genes were selected based on adjusted p-value < 0.05, log2 (fold change) < -1 or > 1;
(ii) expression of representative genes corresponding to BCR signaling that are significantly dysregulated by EC-7072 in CLL cells using a scale representing the per-gene Z-score; and
(iii) expression of genes related to apoptosis regulation that are significantly dysregulated by EC-7072 in CLL cells using a scale representing the per-gene Z-score;
(iii) significantly dysregulated genes involved in NF-κB signaling pathway in CLL cells upon EC-7072 treatment using a scale representing the per-gene Z-score.

Expression of BCR-related genes was analyzed by qPCR in isolated CLL leukemia cells (n = 6) exposed to 200 nM EC-7072 for 6 hours (**Figure 6A**).

KEGG pathway analysis was performed to identify selected relevant pathways for CLL cell homeostasis significantly modulated by EC-7072 (**Figure 6B**)**.**

PBMCs from patients with CLL (n = 8) were treated with 200 nM EC-7072 for 6 hours. Viability was assessed by DiOC₆(3)/PI staining and normalized to the control (DMSO) condition for each individual patient to obtain the mean percentage of viable [DiOC₆(3)⁺] cells (**Figure 6C**).

Expression of relevant genes that regulate apoptosis in CLL was analyzed by qPCR in isolated leukemia cells from patients with CLL (n = 6) exposed to 200 nM EC-7072 for 6 hours, to obtain the mean relative expression to the control (DMSO) control condition (**Figure 6D**).

Western blot analysis of BCL-xL and Noxa in isolated CLL cells (n = 3) treated with 200 nM EC-7072 for the indicated times was performed (**Figure 6E**).

The dysregulation of *BCL2* expression in primary CLL by EC-7072 was evaluated by:
(i) incubating isolated CLL cells (n = 6) from patients with CLL following treatment with EC-7072 (200 nM) for 6 hours and extracting total RNA. Relative expression of *BLC2* was determined by qPCR to obtain the mean relative expression to the control (DMSO) condition (**Figure 6F**);
(ii) incubating isolated CLL cells (n = 6) following treatment with 200 nM EC-7072 for 12 and 18 hours. *BCL2* levels were evaluated by intracellular flow cytometry (**Figure 6G**);
(iii) incubating isolated T cells (n = 3) from patients with CLL following treatment with EC-7072 (200 nM) for 6 hours and extracting total RNA. Relative expression of *BLC2* was determined by qPCR to obtain the mean relative expression to the control (DMSO) condition (**Figure 6H**);
(iv) incubating PBMCs (**Figure 6I**) and isolated T cells (**Figure 6J**) from patients with CLL (n = 3) following treatment with 200 nM EC-7072 for 24 hours. T-cell death and apoptosis were assessed by DiOC₆(3)/PI staining to obtain percentages of viable [DiOC₆(3)⁺] cells and the mean percentages of apoptotic [DiOC₆(3)^{low} PI⁻] and dead [PI⁺] cells.

### 7.2 Results

To elucidate the mechanisms underpinning the cytotoxic effect of EC-7072 against CLL cells, the transcriptional profile of primary leukemia cells from 8 patients with CLL exposed to 200 nM EC-7072 for 6 hours were interrogated. RNA-seq analysis identified 2531 differentially expressed genes in EC-7072-treated compared with control (DMSO) leukemia cells, hence demonstrating that the compound dramatically impacts the transcriptome of CLL cells. Functional profiling of these transcripts revealed that EC-7072 modulates the expression of key mediators of signaling pathways that are paramount for CLL cell homeostasis and survival. Thus, exposure to EC-7072 resulted in a broad downregulation of genes required for functional BCR signaling, including members of the BCR complex (CD79B), BCR-proximal-related kinases (SYK, LYN, PIK3CD) and downstream signaling effectors (PLCG2, CARD11), which was subsequently validated by qPCR (**Figure 6A**)**.** Accordingly, KEGG pathway analysis of the protein-coding genes differentially expressed in CLL cells after EC-7072 treatment revealed an enrichment of multiple cascades engaged by the BCR-dependent signaling, such as NF-kB, JAK/STAT, PI3K/AKT and MAPK pathways, with key roles in regulating gene transcription in CLL cells (**Figure 6B**)**.** In other words, signaling pathways that are engaged downstream of the BCR cascade (including NF-κB, JAK/STAT and MAPK) were found altered in KEGG analysis of the differentially expressed genes, suggesting a profound dysregulation of the BCR signaling pathway in CLL cells brought about by EC-7072. Dysregulation of these signaling networks entailed by impaired BCR-dependent signaling can lead to CLL cell demise by modulating the expression of molecules that govern apoptosis, a process that also scored among the most-enriched pathways in CLL cells upon treatment with EC-7072. Indeed, despite the mithralog not triggering detectable cell death under these experimental settings (6 hours, 200 nM EC-7072) (**Figure 6C**), a marked shift in the expression profile of genes that govern apoptosis was already observed, thereby underscoring the ability of EC-7072 to subvert CLL cell viability (**Figure 6D**). In agreement, Western blotting analysis showed an increase in the levels of the proapoptotic protein Noxa, while the antiapoptotic protein BCL-xL was decreased, in primary leukemia cells exposed to EC-7072 in a time-dependent manner, further supporting the proapoptotic properties of the mithralog in CLL (**Figure 6E**). Among these apoptotic regulators, it is worth mentioning that the gene expression of BCL2, an antiapoptotic protein overexpressed in CLL associated to leukemia cell survival and therapy resistance, was significantly downregulated in primary CLL cells after treatment with EC-7072 (**Figure 6F**). Accordingly, a mild, but significant, decrease in the protein levels of BCL2 was observed on EC-7072-treated CLL cells by intracellular flow cytometry (**Figure 6G**). Conversely, BCL2 expression was not modulated by the compound in T cells isolated from patients with CLL, thereby reinforcing the notion that EC-7072 is highly efficient at inducing leukemia cell apoptosis compared to other immune cell subsets in patients with CLL (**Figures 6H** to **6J**). Thus, consistent with the experiments demonstrating that EC-7072 elicits apoptotic CLL cell death in a caspase-3-dependent manner, the mithralog reprogrammed leukemia cell expression of a set of genes that control apoptosis, including the downregulation of BCL2 gene and protein expression, an antiapoptotic protein highly expressed in CLL cells that is an attractive target for CLL therapies, and a pronounced increase in Noxa levels, a proapoptotic protein commonly upregulated in CLL as a result of treatment with apoptosis-inducing agents.

Leukemia and non-malignant B cells are heavily dependent on the BCR signaling pathway for survival. Signaling through the BCR propagates via phosphorylation of downstream mediators, eventually resulting in constitutive activation of signaling pathways -such as NF-κB, JAK/STAT and MAPK- that sustain leukemia cell viability by, at least in part, upregulating the expression of antiapoptotic members of the BCL2 family of proteins. Consequently, disruption of the BCR pathway by blocking the mediators of the cascade often results in CLL cell death, which is the mainstay of the therapeutic efficacy of targeted agents employed in the treatment of CLL. These RNA-seq studies revealed that EC-7072 may target the BCR signaling pathway at multiple levels in primary CLL cells from patients. Altogether, these results reveal that EC-7072 shapes the transcriptional landscape of primary CLL cells, reprogramming the expression of genes that control cell survival toward a pro-apoptotic state.

### 8. 3.5 EC-7072 inhibits tonic BCR pathway by suppressing the expression and phosphorylation of key signaling nodes

### 8.1 Methodology

The inhibition of the tonic BCR pathway by EC-7072 was evaluated by:
(i) incubating PBMCs from patients with CLL, following treatment with 200 nM EC-7072, for 8 hours. Surface expression of CD79A (**Figure 7A**), CD79B (**Figure 7B**) and IgM (**Figure 7C**) in leukemia cells (n = 5 - 6) was assessed by flow cytometry and the MFI was normalized to the control (DMSO) condition;
(ii) incubating isolated leukemia cells from patients with CLL (n = 10), following treatment with EC-7072 (200 nM), for 8 hours. Phosphorylation levels of p-SYK (**Figure 7D**), p-BTK (**Figure 7E**) and p-PLCy2 (**Figure 7F**) proteins and p-ERK1/2 (**Figure 7G**), p-AKT (**Figure 7H**), p-p65 NPκB (**Figure 7I**) and p-STAT3 (**Figure 7J**) were evaluated by phosphoflow and the MFI was normalized to the control (DMSO) from each individual patient;
(iii) incubating PBMCs from patients with CLL (n = 8), following treatment with EC-7072 (200 nM), for 8 hours. Viability was determined by DiOC₆(3)/PI staining and normalized to the control (DMSO) condition for each individual patient to obtain the mean percentage of viable [DiOC₆(3)⁺] cells (**Figure 7K**);
(iv) incubating isolated CLL cells (n = 4) stimulated with CD40L (100 ng/mL) or IL-6 (40 ng/mL) for 15 minutes and detecting respective phosphorylation levels of p65 NF-κB (**Figure 7L**) and STAT3 (**Figure 7M**) by phosphoflow to obtain the mean MFI normalized to the control (DMSO) condition for each individual patient;
(v) incubating isolated leukemia cells from 3 patients with CLL, following treatment with 200 nM EC-7072, for 3, 8 and 12 hours. Protein lysates were immunoblotted to detect the phosphorylated (p-LYN, p-PLCγ2, p-STAT3 and p-ERK1/2) and total protein (LYN, PLCγ2, STAT3 and ERK1/2) levels (**Figure 7N**);
(vi) incubating isolated leukemia cells from 3 patients with CLL, following treatment with 200 nM EC-7072, for 3, 8 and 12 hours and immunoblotting of protein lysates to detect p-LYN, LYN, p-PLCγ2, PLCγ2, p-STAT3, STAT3, p-ERK1/2 and ERK1/2. Levels of phosphorylated and total proteins were quantified by densitometric analysis and the signal intensities were normalized to the control (GAPDH, i.e. 0 hours) condition (**Figure 7O**) and signal intensities corresponding to phosphorylated proteins were normalized to their respective total amount for each time point to obtain the mean value of the densitometry of Western blot analyses of primary CLL cells from 3 different patients normalized to the control (0 hours) condition (**Figure 7P**);
(vii) treating CLL-derived MEC-1 cells with EC-7072 (200 nM) at 0-, 3-, 8- and 12-hour time points followed by extracting protein lysates. Phosphorylated (p)- and total STAT3 were immunoblotted (**Figure 7Q**)**.**

### 8.2 Results

RNA-seq analyses in Section 7 (above) suggest that EC-7072 targets the BCR signaling axis by suppressing the expression of several components that operate at different levels of the cascade. Activation of the BCR signaling pathway is initiated by engagement of the BCR, a multimeric complex composed of a membrane immunoglobulin and the heterodimer CD79A/B (Igα/β). In agreement with the transcriptomic studies it was found that, despite the levels of CD79A remaining unchanged after treatment, EC-7072 significantly decreased CD79B and IgM CLL cell surface expression (**Figures 7A** to **7C**). In particular, exposure to the mithralog resulted in a broad downregulation of protein-coding genes of components of the BCR complex (*CD79B*), BCR-proximal kinases (*SYK, LYN, PIK3CD*) and downstream signaling effectors (*PLCG2, CARD11*).

Initial stimulation at the BCR complex leads to a phosphorylation cascade of intracellular mediators that amplifies the BCR signal. Therefore, upon demonstration that EC-7072 downregulates expression of BCR components, evaluation of the phosphorylation of BCR signaling nodes by phosphospecific flow cytometry analysis was carried out. Basal levels of phosphorylation of signaling mediators of the BCR cascade were highly heterogeneous in primary leukemia cells from patients with CLL. Nevertheless, as shown in **Figures 7D** to **7F**, these experiments revealed a significant reduction of the levels of phosphorylated (p)-SYK, p-BTK and p-PLCγ2 in isolated leukemia cells after 8 hours of incubation with EC-7072, a treatment that does not result in noticeable CLL cell death (**Figure 7K**)**.** The activity of these proximal signaling nodes is in turn required for stimulation of downstream pathways that sustain B-cell survival. Accordingly, a significant downregulation was registered in the phosphorylation levels of the effector kinase ERK1/2 and the transcription factors p65 NF-κB and STAT3 (**Figures 7G** to **7K**) upon exposure to EC-7072, which is in line with the results of RNA-sequencing analysis. No marked changes were detected in the amounts of p-AKT, suggesting that the effect of EC-7072 on leukemia cells is not likely to involve modulation of AKT phosphorylation (**Figures 7G** to **7K**)**.** As expected, treatment with recombinant CD40L or IL-6 strongly increased the basal phosphorylation levels of p65 NF-κB and STAT3 respectively, hence supporting that the phosphoflow analyses were reliable and robust (**Figures 7L** and **7M**). In agreement with these results, a time-dependent reduction of p-LYN, p-PLCγ2 and p-ERK1/2 was also evidenced by Western blotting (**Figures 7N** and **7O**)**.** Likewise, the levels of p-STAT3, albeit low, diminished upon exposure to EC-7072 (**Figures 7N** and **7O**), which was further confirmed in the CLL-derived cell line MEC-1 (**Figure 7Q**)**.** Concomitantly, EC-7072 was able to downregulate the total amount of LYN, PLCγ2 and STAT3 in CLL cells, further reinforcing the broad transcriptional reprogramming of primary leukemia cells from patients with CLL upon exposure to the compound (**Figures 7N** and **7O**)**.** Of note, except for ERK1/2, the decrease in the phosphorylation levels of these proteins clearly correlated with the observed reduction of their total amount, suggesting that EC-7072 might be mainly hindering BCR signaling via downregulation the expression of key signaling mediators of the pathway (**Figure 7P**)**.**

Altogether, these data suggest that EC-7072 impairs tonic BCR signaling in primary CLL cells by suppressing the expression and function of multiple key BCR pathway nodes.

### 9. Activation of the BCR partially abrogates EC-7072-induced cell death of primary CLL cells

### 9.1 Methodology

The partial abrogation of EC-7072-induced cell death of primary CLL cells by activation of the BCR was evaluated by:
(i) incubating PBMCs from patients with CLL (n = 15), following treatment with EC-7072 (200 nM) in combination with α-IgM (10 µg/mL), for 24 hours. Leukemia cell viability was determined by cytofluorometric assessment of DiOC₆(3)/PI staining and the percentage of viable [DiOC₆(3)⁺] cells was normalized to their respective control (DMSO) condition for each individual patient (**Figure 8A**);
(ii) incubating isolated leukemia cells from patients with CLL (n = 10), following treatment with EC-7072 (200 nM), for 8 hours, followed by stimulation with α-IgM (10 µg/mL) for 15 minutes. Levels of phosphorylated SYK, BTK and PLCγ2 were analyzed by phosphoflow and the MFI was normalized to the control (DMSO) condition (**Figure 8B**);
(iii) incubating isolated leukemia cells from patients with CLL, following treatment with EC-7072 (200 nM), for 8 hours, followed by stimulation with α-IgM (10 µg/mL) for 15 minutes. Levels of phosphorylated ERK1/2, AKT (n = 10), p65 NF-κB and STAT3 (n = 6) were analyzed by phosphoflow and the MFI was normalized to the control (DMSO) condition (**Figure 8C**);
(iv) incubating isolated leukemia cells from patients with CLL (n = 4), following treatment with EC-7072 (200 nM) in combination with α-IgM (10 µg/mL), for 6 hours and extracting total RNA. Relative expression of *BCL2* was analyzed by qPCR and the mRNA relative expression was normalized to the control (DMSO) condition (**Figure 8D**)**.**

### 9.2 Results

Based on the observation that EC-7072 subverts BCR signaling in CLL cells, it was investigated whether this suppressive role underpins the antileukemic activity of the mithralog. It was reasoned that activation of the BCR may counteract the cytotoxic effect of EC-7072 against primary CLL cells from patients. As shown in **Figure 8A****,** BCR stimulation with anti-IgM antibodies significantly rescued leukemia cell survival in the presence of EC-7072 and BCR crosslinking strongly induced the phosphorylation of downstream mediators of the pathway (**Figures 8B** and **8C**)**.** Accordingly, BCR stimulation resulted in increased levels of phosphorylated SYK and PLCγ2 in CLL cells treated with EC-7072 compared to unstimulated CLL cells exposed to the mithralog (**Figure 8B**)**.** Furthermore, the enhanced phosphorylation of ERK1/2, AKT, p65 NF-κB and STAT3 ensued by treatment with anti-IgM was not significantly reverted by EC-7072 (**Figure 8C**)**.** Of note, BCR activation was able to restore the expression of *BCL2* to basal levels in isolated CLL cells treated with the mithralog, further supporting the pro-survival role of anti-IgM by antagonizing the pro-apoptotic activity of EC-7072 against leukemia cells (**Figure 8D**)**.**

Indeed, the functional relevance of the impact of the mithralog on the transcriptional landscape of primary CLL cells was further evidenced by demonstration of reduced levels of phosphorylated signaling nodes and pathways downstream of the BCR, suggesting that EC-7072 may hamper constitutive tonic activation of BCR-dependent signaling cascades. Such an effect is in line with the finding that EC-7072 rapidly reduces the surface expression of BCR subunits (CD79B and IgM). It was also found that BCR stimulation is able to antagonize the repression of downstream signaling nodes and, noteworthy, significantly counteract the apoptotic cell death triggered by EC-7072, which was accompanied by upregulation of BCL2 gene expression, further supporting that the antileukemic activity of the compound may involve modulation of the basal BCR signaling status and the proapoptotic/antiapoptotic balance in CLL cells.

Overall, these experiments show that stimulation of the BCR, as illustrated by the enhanced phosphorylation of BCR signaling downstream mediators detected, counteracts the cytotoxicity of EC-7072 against primary CLL cells, hence supporting that the mithralog negatively modulates tonic BCR signaling. Thus, overstimulation of the BCR signaling by antibody-mediated receptor crosslinking was able to largely counteract the antileukemic activity of EC-7072.

This work was supported by a grant from the Spanish Instituto de Salud Carlos III (PI16/01485) and FEDER European Union, a grant from IDEPA (B74079153) and a project granted by Sociedad Asturiana de Hematología y Hemoterapia.

## Claims

1. A compound of Formula (I), 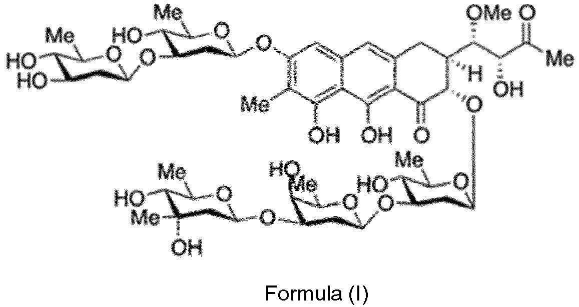 or a pharmaceutically acceptable salt, co-crystal or solvate thereof, for use in treatment of chronic lymphocytic leukemia.

2. The compound for use according to claim 1, wherein said compound of Formula (I), or a salt, co-crystal or solvate thereof, is administered simultaneously with, separately from or sequentially to the administration of at least one chemotherapeutic agent selected from idelalisib or fludarabine, or a salt, co-crystal or solvate thereof.

3. The compound for use according to claim 2, wherein the chemotherapeutic agent is idelalisib and the molar ratio of the compound of the Formula (I) to idelalisib is from 1:3 to 1:300.

4. The compound for use according to claim 2, wherein the chemotherapeutic agent is fludarabine and the molar ratio of the compound of the Formula (I) to fludarabine is from 1:7 to 1:380.

5. A composition comprising:
(a) a compound of Formula (I), 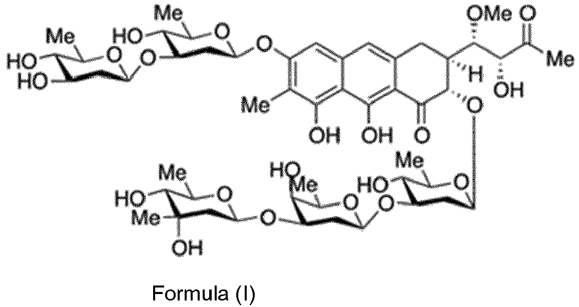 or a pharmaceutically acceptable salt, co-crystal or solvate thereof; and
(b) at least one chemotherapeutic agent selected from idelalisib or fludarabine, or a salt, co-crystal or solvate thereof.

6. The composition according to claim 5, wherein the chemotherapeutic agent is idelalisib and the molar ratio of the compound of the Formula (I) to idelalisib is from 1:3 to 1:300.

7. The composition according to claim 5, wherein the chemotherapeutic agent is fludarabine and the molar ratio of the compound of the Formula (I) to fludarabine is from 1:7 to 1:380.

8. A composition according to any of claims 5 to 7 for use as a medicament.

9. A composition according to any of claims 5 to 7 for use in treatment of chronic lymphocytic leukemia.

10. A method for producing a composition according to any of claims 5 to 7, which comprises mixing:
(i) a compound of Formula (I), 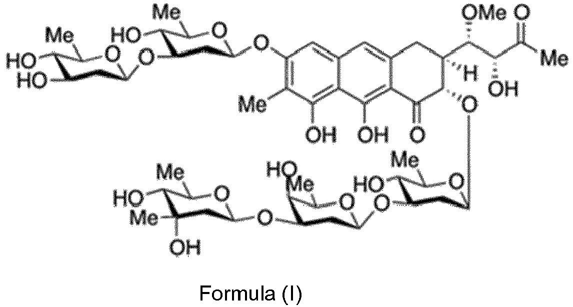 or a pharmaceutically acceptable salt, co-crystal or solvate thereof; and
(ii) at least one chemotherapeutic agent selected from idelalisib or fludarabine, or a salt, co-crystal or solvate thereof.

11. The method according to claim 10, wherein the chemotherapeutic agent is idelalisib and the molar ratio of the compound of the Formula (I) to idelalisib is from 1:10 to 1:100.

12. The method according to claim 10, wherein the chemotherapeutic agent is fludarabine and the molar ratio of the compound of the Formula (I) to fludarabine is from 1:10 to 1:100.

13. A kit-of-parts for use in treatment of chronic lymphocytic leukemia, comprising:
(a) a compound of Formula (I), 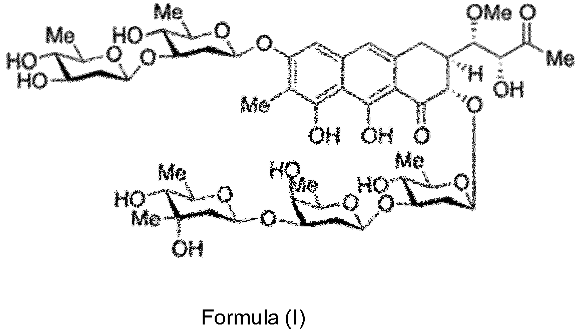 or a pharmaceutically acceptable salt, co-crystal or solvate thereof; and
(b) at least one chemotherapeutic agent selected from idelalisib or fludarabine, or a salt, co-crystal or solvate thereof.

14. The kit-of-parts according to claim 13, wherein the chemotherapeutic agent is idelalisib and the molar ratio of the compound of the Formula (I) to idelalisib is from 1:3 to 1:300.

15. The kit-of-parts according to claim 13, wherein the chemotherapeutic agent is fludarabine and the molar ratio of the compound of the Formula (I) to fludarabine is from 1:7 to 1:380.
